# EUROPEAN PATENT SPECIFICATION

(11) **EP 4 044 943 B1**
(45) Date of publication and mention of the grant of the patent: **07.05.2025**
(21) Application number: 20877676.5
(22) Date of filing: 16.10.2020
(51) Int. Cl.: A61B 17/3207

(54) **ATHERECTOMY CATHETER WITH SHAPEABLE DISTAL TIP**
ATHEREKTOMIEKATHETER MIT FORMBARER DISTALER SPITZE
CATHÉTER D'ATHÉRECTOMIE AVEC POINTE DISTALE FORMABLE

(30) Priority: 18.10.2019 US 201962923368 P; 13.05.2020 US 202063024306 P
(43) Date of publication of application: 24.08.2022
(73) Proprietor: Avinger, Inc., Redwood City, CA 94063 (US)
(72) Inventor: PATEL, Himanshu N., Redwood City, CA 94063 (US); ZUNG, Michael, Redwood City, CA 94063 (US)
(74) Representative: Potter Clarkson
(86) International application number: PCT/US2020/056072
(87) International publication number: WO 2021/076957

(56) References cited:
- EP-A1- 3 446 648
- WO-A1-2018/006041
- US-A1- 2003 125 757
- US-A1- 2003 125 757
- US-A1- 2016 199 092
- US-A1- 2016 310 700
- US-A1- 2016 374 718
- US-A1- 2019 209 206
- US-B1- 6 599 296

## Description

### CROSS REFERENCE TO RELATED APPLICATIONS

This claims priority to U.S. Provisional Patent Application No. 63/024,306, filed on May 13, 2020, entitled "ATHERECTOMY CATHETER WITH SHAPEABLE DISTAL TIP," and to U.S. Provisional Patent Application No. 62/923,368, filed on October 18, 2019, entitled "ATHERECTOMY CATHETER WITH SHAPEABLE DISTAL TIP".

This application may also be related to International Application No. PCT/US2017/040431, filed on June 30, 2017, entitled "ATHERECTOMY CATHETER WITH SHAPEABLE DISTAL TIP," which claims priority to U.S. Provisional Patent Application No. 62/357,173, filed on June 30, 2016, entitled "ATHERECTOMY CATHETER WITH SHAPEABLE DISTAL TIP".

This application may also be related to International Application No. PCT/US2019/028415, filed on April 19, 2019, entitled "OCCLUSION-CROSSING DEVICES," which claims priority to U.S. Provisional Patent Application No. 62,768,769, filed on November 16, 2018, entitled "OCCLUSION-CROSSING DEVICES," and to U.S. Provisional Patent Application No. 62/660,185, filed April 19, 2018, entitled "OCCLUSION-CROSSING DEVICES".

### FIELD

Described herein are devices for treatment of an occluded body lumen, such as for the removal of occlusive materials from blood vessels. In particular, described herein are atherectomy catheters that are adapted to easily maneuver against tissue and plaque buildup within vessels for debulking.

### BACKGROUND

Atherosclerosis is disease in which accumulation of atheromatous materials builds up inside a person's arteries. Atherosclerosis occurs as part of the natural aging process, but may also occur due to a person's diet, hypertension, vascular injury, heredity, and so forth. Atherosclerosis can affect any artery in the body, including arteries in the heart, brain, arms, legs, pelvis, and kidneys. Atherosclerosis deposits may vary in their properties as well. Some deposits are relatively soft, other types may be fibrous, some are calcified, or a combination of all three. Based on the location of the plaque accumulation, different diseases may develop. For example, coronary heart disease occurs when plaque builds up in the coronary arteries, which supply oxygenated blood to the heart. If plaque buildup blocks the carotid artery, arteries located on each side of the neck that supply oxygen to the brain, a stroke may be the result.

Atherosclerosis may be treated in a number of ways including medication, bypass surgery, and catheter-based approaches. Atherectomy procedures involve excising or dislodging materials that block a blood vessel. Many atherectomy catheters typically have a substantially straight central axis. However, atherectomy catheters having a straight profile may be difficult to maneuver close enough to the inner surface of the arterial walls to remove all plaque buildup. Moreover, plaque removal can be complicated with such straight profile catheters when plaque formations accumulate in the curves and more tortuous portions of an artery.

The atherectomy catheters described herein address some of these challenges.

### SUMMARY OF THE DISCLOSURE

The invention is defined by independent claim 1. Further embodiments are defined by the dependent claims. No surgical methods are claimed.

Described herein are atherectomy catheters for use in vessels. The catheters can include a rotatable cutter within a catheter. The shape of the catheter can be configured to aid optimal positioning of the cutter, for example during a cutting procedure. In some cases, the cutter may be extended through a window of the catheter upon translation of the cutter within the catheter. In some cases, the cutter is retractable into the catheter.

In one embodiment, an atherectomy catheter for use in a vessel includes an elongate catheter body and an annular cutter. The elongate catheter body includes a fixed jog section with a pre-set curvature and a flexible section that has a greater flexibility than a remainder of the elongate catheter body. The fixed jog section and flexible section are formed of a frame including a plurality of circumferential slits therein.

This and other embodiments can include one or more of the following features. The frame in the fixed jog section can further include a longitudinal spine extending therethrough that does not have slits. The atherectomy catheter can further include a cutting window through which the annular cutter extends. The cutting window can be positioned distal of the fixed jog section and the flexible section so as to urge the cutter into the vessel. The atherectomy catheter can further include at least one laminating layer positioned over or under the frame of the fixed jog section. The laminating layer can be made of a polymer. The frame can be made of metal. The plurality of circumferential slits can be arranged in a repeating pattern. The fixed jog section can form an angle of 130° to 160° in the elongate catheter body. The frame can further include an annular spine without slits that extends between the fixed jog section and the flexible section. The flexible section can be configured to passively bend to angles of 130°-160°.

In general, in one embodiment, an atherectomy catheter for use in a vessel includes an elongate catheter body, an annular cutter, and curved portion in the elongate catheter body. The curved portion can have any of a number of shapes, such as an s-shape. The curved portion includes a frame having a plurality of annular spines connected together by a longitudinal proximal spine and a longitudinal distal spine. The longitudinal proximal spine is positioned approximately 180 degrees away from the longitudinal distal spine.

This and other embodiments can include one or more of the following features. The plurality of annular spines can include a first annular spine, a second annular spine, and a third annular spine. The longitudinal proximal spine can connect the first annular spine and the second annular spine, and the longitudinal distal spine can connect the second annular spine and the third annular spine. The atherectomy catheter can further include a cutting window through which the annular cutter extends. The cutting window can be positioned distal of the curved portion and on an outer circumference of the s-shaped curve so as to urge the cutter into the vessel. The s-shaped curved portion can be configured to be activated by pulling or pushing on a shaft of the atherectomy catheter. The atherectomy catheter can further include at least one laminating layer positioned over or under the frame. The laminating layer can be made of a polymer. The frame can be made of metal. The distal longitudinal spine can be positioned adjacent to an exposed portion of the cutter. The distal longitudinal spine can be on a same side of the elongate catheter body as the exposed portion of the cutter. The longitudinal proximal spine can form a first angle, and the longitudinal distal spine can form a second angle. The first and second angles can extend in opposite directions, and the first angle can be between 140 and 160 degrees and the second angle can be between 140 and 160 degrees. A distal-most spine of the plurality of spines can include a beveled distal edge. The atherectomy catheter can further include a nosecone configured to pivot away from the elongate body to expose the cutter. The bevel can be configured to provide space for the nosecone to pivot.

In general, in one embodiment, an atherectomy catheter for use in a vessel includes an elongate catheter body, an annular cutter, and an s-shaped curved portion in the elongate catheter body. The curved portion includes a frame having a proximal section and a distal section. The proximal section has a plurality of circumferential proximal slits and a longitudinal proximal spine without slits, and the distal section having a plurality of circumferential distal slits and a longitudinal distal spine without slits. The longitudinal proximal spine is positioned approximately 180 degrees away from the longitudinal distal spine.

This and other embodiments can include one or more of the following features. The atherectomy catheter can further include a cutting window through which the annular cutter extends. The cutting window can be positioned distal of the distal section and on an outer circumference of the s-shaped curve so as to urge the cutter into the vessel. The s-shaped curved portion can be configured to be activated by pulling or pushing on a shaft of the atherectomy catheter. The atherectomy catheter can further include at least one laminating layer positioned over or under the frame. The laminating layer can be made of a polymer. The frame can be made of metal. The plurality of circumferential proximal slits can be arranged in a first repeating pattern, and the plurality of circumferential distal slits can be arranged in a second repeating pattern. The first repeating pattern and the second repeating pattern can be circumferentially offset from one another. The distal longitudinal spine can be positioned adjacent to an exposed portion of the cutter. The distal longitudinal spine can be on a same side of the elongate catheter body as the exposed portion of the cutter. The proximal section can form a first angle, and the distal section forms a second angle. The first and second angles can extend in opposite directions, and the first angle can be between 140 and 160 degrees and the second angle can be between 140 and 160 degrees. The frame can further include an annular spine without slits extending between the proximal section and the distal section.

In general, in one embodiment, an atherectomy catheter for use in a vessel includes an elongate catheter body, an annular cutter, and an s-shaped curved portion in the elongate catheter body. The curved portion includes a frame having a proximal section and a distal section. The proximal section has a plurality of circumferential proximal slits and a longitudinal proximal spine without slits, and the distal section having a plurality of circumferential distal slits and a longitudinal distal spine without slits. The longitudinal proximal spine is positioned approximately 180 degrees away from the longitudinal distal spine, and the circumferential slits are in a tongue and groove formation.

In some examples, an atherectomy device includes: a catheter including a distal nosecone fixedly coupled to a proximal flexible section and a cutter window between the distal nosecone and the proximal flexible section, wherein an extent of curvature of the proximal flexible section is adjustable; and a cutter coupled to a rotatable driveshaft within the catheter, wherein proximal movement of the cutter and the rotatable driveshaft causes the cutter to tilt in a first direction and to extend though the cutter window, wherein distal movement of the cutter and the rotatable driveshaft causes the cutter to tilt in a second direction opposite the first direction and to retract within the catheter. The extent of curvature of the proximal flexible section can be adjustable based on an amount of force applied to the cutter and the rotatable driveshaft in a proximal direction. Initial proximal movement of the cutter and the rotatable driveshaft can cause the cutter to tilt in the first direction and extend through the cutter window, where further proximal movement of the cutter and the rotatable driveshaft can cause the flexible section to bend. Likewise, distal movement of the cutter and the rotatable driveshaft can cause the flexible section to straighten from a bent state. The proximal flexible section can be configured to bend to an s-shape. The proximal flexible section can be configured to bend incrementally based on an amount of compression on the proximal flexible section via proximal movement of the cutter and the rotatable driveshaft. The cutter can include a ledge that is configured to slide along an edge of an inner surface of the catheter to move the cutter radially and extend the cutter through the cutting window. The edge can be sloped with respect to an axis perpendicular to the longitudinal axis of the distal nosecone. The edge can be configured to tilt the cutter with respect to the nosecone when the ledge of the cutter slides along the edge. The edge can be on a bushing of the catheter. The cutter window can be on a side of the catheter.

In some examples, an atherectomy device includes: a catheter including a nosecone fixedly coupled to a elongate body at a fixed bend of the catheter, wherein the catheter includes a cutter window on a convex side of the fixed bend; and a cutter coupled to a rotatable driveshaft within the catheter, wherein proximal movement of the cutter and the rotatable driveshaft causes the cutter to tilt in a first direction and to extend though the cutter window, wherein distal movement of the cutter and the rotatable driveshaft causes the cutter to tilt in a second direction opposite the first direction and to retract within the catheter. The cutting window can be distally located along the catheter with respect to the fixed bend. The cutter can be configured to move radially when extending through the cutting window. At least a portion of a cutting edge of the cutter can correspond to a most prominent point along the convex side of the fixed bend when the cutter is extended through the window. The cutter can be configured to transition between an active mode and a passive mode, wherein a cutting edge of the cutter extends through the window in the active mode, and wherein the cutting edge of the cutter is retracted within the catheter in the passive mode. The cutter can be substantially parallel to the nosecone in the active mode and substantially parallel to the elongate body in the passive mode. The cutting edge of the cutter can be held in the passive mode by a detent that requires a threshold translational force applied to the rotatable driveshaft to release the detent and transition the cutter from the passive mode. The cutter can include an annular groove that provides clearance for an inner surface of the catheter. The cutter can be rotatable when in the active mode and the passive mode. The cutter can include an imaging sensor configured to collect images outside of the catheter while the cutter is in the active mode and the passive mode. The catheter can include one or more openings configured to align with the imaging sensor and act as a location marker for the imaging sensor when the cutter is in the active mode. The fixed bend can have an angle ranging from 1 degree to 30 degrees. A central axis of the cutter can be at an angle ranging from 1 degree to 30 degrees with respect to a central axis of the distal nosecone when the cutter is extended through the cutting window. The cutter can include a ledge that is configured to slide along an edge within the lumen of the catheter to move the cutter radially and extend the cutter through the cutting window upon proximal movement of the cutter and the rotatable driveshaft. A lumen of the catheter can define a first channel and a second channel, wherein a sloped edge within the lumen is configured to urge the cutter from the first channel to the second channel upon proximal movement of the cutter and the rotatable driveshaft. The cutter can be configured to move distally to pack tissue into the distal nosecone. The cutter can be configured to transition between being parallel to the nosecone and parallel to the elongate body. The elongate body can include a flexible section proximally located relative to the fixed bend, wherein an extent of curvature of the proximal flexible section is adjustable. The flexible section can be configured to take on an s-shaped curved shape upon further proximal movement of the cutter and rotatable driveshaft within the catheter.

In some examples, an atherectomy device includes: a catheter including a distal nosecone fixedly coupled to a flexible section and a cutter window between the distal nosecone and the flexible section, wherein the flexible section includes a longitudinal spine on a side of the flexible section; and a cutter coupled to a rotatable driveshaft within the catheter, wherein proximal movement of the cutter and the rotatable driveshaft causes the cutter to tilt in a first direction and to extend though the cutter window, wherein a force applied to the rotatable shaft in a proximal direction causes the flexible section to bend away from the longitudinal spine and to take on a curvature, and wherein distal movement of the cutter and the rotatable driveshaft causes the cutter to tilt in a second direction opposite the first direction and to retract within the cutter window. An extent of curvature of the flexible section can be adjustable based on an amount of force applied to the cutter and the rotatable driveshaft in a proximal direction. The proximal flexible section can be configured to bend to an s-shape. The flexible section can be configured to bend incrementally based on an amount of compression on the flexible section via proximal movement of the cutter and the rotatable driveshaft. The cutter can include a ledge that is configured to slide along an edge of an inner surface of the catheter to move the cutter radially and extend the cutter through the cutter window. The edge can be sloped with respect to an axis perpendicular to the longitudinal axis of the distal nosecone. The edge can be configured to tilt the cutter with respect to the nosecone when the ledge of the cutter slides along the edge. The edge can be on a bushing of the catheter. The atherectomy device can further include a handle at a proximal end of the catheter, wherein the handle includes a lock configured to lock the flexible section in a curved shape having a selected extent of curvature. The lock can be configured to allow a user to choose a locked position of the driveshaft with respect to the catheter by 0.026 inches (to be considered for the whole description: 1 inch ≈ 25.4 mm) or less. The lock can include a slider button that is configured to slide distally and proximally. Sliding the slider button proximally can increase the curvature of the flexible section. The slider button can include teeth that are configured to engage with corresponding teeth within the handle to lock an axial position of the driveshaft with respect to the catheter. The handle can include a spring that applies pressure to the slider button to keep the teeth of the slider button engaged with the corresponding teeth within the handle. The slider button can be configured to compress the spring when a user presses on the slider button to disengage the teeth of the slider button from the corresponding teeth within the handle. The handle can include a spine joint that allows axial translation of the slider button with respect to the driveshaft while allowing the driveshaft to rotate with respect to the slider button. The flexible section can include a first portion axially adjacent to a second portion, the first portion having a first longitudinal spine and the second portion having a second longitudinal spine, wherein the first longitudinal spine and second longitudinal spine are on opposing sides of the flexible section, and wherein the force applied to the rotatable shaft in the proximal direction causes the first portion to bend laterally away from the first longitudinal spine and the second portion to bend laterally away from the second longitudinal spine.

In some examples, an atherectomy device includes: a catheter including a nosecone fixedly coupled to an elongate body at a fixed bend of the catheter, wherein the catheter includes a cutter window on a convex side of the fixed bend; and a cutter coupled to a rotatable driveshaft within the catheter, wherein proximal movement of the cutter and the rotatable driveshaft causes the cutter to tilt in a first direction and to extend though the cutter window, wherein distal movement of the cutter and the rotatable driveshaft causes the cutter to tilt in a second direction opposite the first direction and to retract within the catheter. The cutter window can be distally located along the catheter with respect to the fixed bend. The cutter can be configured to move radially when extending through the cutter window. At least a portion of a cutting edge of the cutter can correspond to a most prominent point along the convex side of the fixed bend when the cutter is extended through the cutter window. The cutter can be configured to transition between an active mode and a passive mode, wherein a cutting edge of the cutter extends through the cutter window in the active mode, and wherein the cutting edge of the cutter is retracted within the catheter in the passive mode. The cutter can be substantially parallel to the nosecone in the active mode and substantially parallel to the elongate body in the passive mode. The cutting edge of the cutter can be held in the passive mode by a detent that requires a threshold translational force applied to the rotatable driveshaft to release the detent and transition the cutter from the passive mode. The cutter can be rotatable when in the active mode and the passive mode. The cutter can include an imaging sensor configured to collect images outside of the catheter while the cutter is in the active mode and the passive mode. The catheter can include one or more openings configured to align with the imaging sensor and act as a location marker for the imaging sensor when the cutter is in the active mode. The fixed bend can have an angle ranging from 1 degree to 30 degrees. A central axis of the cutter is at an angle ranging from 1 degree to 30 degrees with respect to a central axis of the distal nosecone when the cutter is extended through the cutter window. The cutter can include a ledge that is configured to slide along an edge within the lumen of the catheter to move the cutter radially and extend the cutter through the cutter window upon proximal movement of the cutter and the rotatable driveshaft. A lumen of the catheter defines a first channel and a second channel, wherein a sloped edge within the lumen is configured to urge the cutter from the first channel to the second channel upon proximal movement of the cutter and the rotatable driveshaft. The cutter can be configured to move distally to pack tissue into the distal nosecone. The cutter can be configured to transition between being parallel to the nosecone and parallel to the elongate body. The elongate body can include a flexible section proximally located relative to the fixed bend, wherein an extent of curvature of the flexible section is adjustable based on an extent of proximal movement of the cutter and the rotatable driveshaft relative to the catheter. The flexible section can be configured to take on an s-shaped curve upon proximal movement of the cutter and rotatable driveshaft within the catheter. The atherectomy device can further include a handle at a proximal end of the catheter, wherein the handle includes a lock configured to lock the flexible section in a curved shape. The lock can be configured to allow a user to choose a locked position of the driveshaft with respect to the catheter by 0.026 inches or less. The lock can include a slider button that is configured to slide distally and proximally. Sliding the slider button proximally can increase the curvature of the flexible section. The handle can include a spine joint that allows axial translation of the slider button with respect to the driveshaft while allowing the driveshaft to rotate with respect to the slider button. The flexible section can include a first portion axially adjacent to a second portion, the first portion having a first longitudinal spine and the second portion having a second longitudinal spine, wherein the first longitudinal spine and second longitudinal spine are on opposing sides of the flexible section, and wherein the force applied to the rotatable shaft in the proximal direction causes the first portion to bend laterally away from the first longitudinal spine and the second portion to bend laterally away from the second longitudinal spine. The elongate body can include a flexible section proximally located relative to the fixed bend, the flexible section including a first portion axially adjacent to a second portion, the first portion having a first longitudinal spine and the second portion having a second longitudinal spine, wherein the first longitudinal spine and second longitudinal spine are on opposing sides of the flexible section. Further proximal movement of the rotatable shaft can cause the flexible section to compress such that the first portion bends laterally away from the first longitudinal spine and the second portion bends laterally away from the second longitudinal spine, thereby causing the flexible section to take on an s-shape. The fixed bend can have an angle ranging from about 1°-30°.

In some examples, a method of using an atherectomy device, the atherectomy device including a cutter coupled to a rotatable driveshaft within a catheter, the catheter having a distal nosecone fixedly coupled to a flexible section and a cutter window between the distal nosecone and the flexible section, the method includes: moving the rotatable driveshaft proximally within the catheter to cause the cutter to tilt in a first direction and extend through the cutter window; moving the rotatable driveshaft further proximally within the catheter to cause the flexible section to bend away from a longitudinal spine of the flexible section such that the flexible section takes on a curvature; and moving the rotatable driveshaft distally within the catheter to cause the cutter to tilt in a second direction opposite the first direction and to retract within the catheter. The method can further include moving the rotatable driveshaft distally within the catheter to cause the flexible section to straighten. Moving the rotatable driveshaft distally can include sliding a slider button on a handle of the atherectomy device distally, wherein moving the rotatable driveshaft proximally includes sliding the slider button proximally. The method can further include sliding a slider button on a handle of the atherectomy device proximally to increase the curvature of the flexible section. The method can further include selecting an extent of curvature of the flexible section by controlling distal and proximal movement of the slider button. The method can further include locking the flexible section in a curved shape by a selected curvature using a handle at a proximal end of the catheter. Moving the rotatable driveshaft proximally to extend the cutter through the cutter window can include causing a ledge of the cutter to slide along an edge of an inner surface of the catheter to move the cutter radially and extend the cutter through the cutter window. The edge can be sloped with respect to an axis perpendicular to the longitudinal axis of the distal nosecone. The edge can be configured to tilt the cutter with respect to the nosecone when the ledge of the cutter slides along the edge. The cutter window can be on a side of the catheter. The flexible section can include a first portion axially adjacent to a second portion, the first portion having a first longitudinal spine and the second portion having a second longitudinal spine, wherein the first longitudinal spine and second longitudinal spine are on opposing sides of the flexible section, and wherein moving the rotatable driveshaft further proximally causes the first portion to bend laterally away from the first longitudinal spine and the second portion to bend laterally away from the second longitudinal spine, thereby causing the flexible section to take on an s-shape. The method can further include rotating the rotatable driveshaft while capturing images outside of the catheter using an imaging sensor coupled to the rotatable driveshaft. The method can further include cutting tissue outside of the catheter by rotating the rotatable driveshaft.

In some examples, a method of using an atherectomy device, the atherectomy device including a cutter coupled to a rotatable driveshaft within a catheter, the catheter including a nosecone fixedly coupled to an elongate body at a fixed bend of the catheter, the method includes: moving the rotatable driveshaft proximally within the catheter to cause the cutter to tilt in a first direction and to extend though the cutter window; and moving the rotatable driveshaft distally within the catheter to cause the cutter to tilt in a second direction opposite the first direction and to retract the cutter within the cutter window. The fixed bend can have an angle ranging from about 1°-30°. The cutter can move radially with respect to the catheter when extending through the cutter window. At least a portion of a cutting edge of the cutter can correspond to a most prominent point along the convex side of the fixed bend when the cutter is extended through the cutter window. A cutting edge of the cutter can extend through the cutter window in an active mode, and wherein the cutting edge of the cutter is retracted within the catheter in a passive mode. The cutter can be substantially parallel to the nosecone in the active mode and substantially parallel to the elongate body in the passive mode. The cutting edge of the cutter can be held in the passive mode by a detent, the method further comprising applying a threshold translational force to the rotatable driveshaft to release the detent and transition the cutter from the passive mode. The cutter can be rotatable when in the active mode and the passive mode. The method can further include collecting images outside of the catheter using an imaging sensor coupled to the rotatable driveshaft. The catheter can include one or more openings configured to align with the imaging sensor and act as a location marker for the imaging sensor. Moving the rotatable driveshaft proximally to extend the cutter through the cutter window can include causing a ledge of the cutter to slide along an edge of an inner surface of the catheter to move the cutter radially and extend the cutter through the cutter window. The method can further include moving the cutter distally to pack tissue into the nosecone. Moving the rotatable driveshaft proximally within the catheter to cause the cutter to tilt in the first direction and to extend though the cutter window can include transitioning the cutter between being parallel to the nosecone and parallel to the elongate body. The elongate body can include a flexible section proximally located relative to the fixed bend, the method further comprising moving the rotatable driveshaft further proximally within the catheter to cause the flexible section to bend. The method can further include adjusting a curvature of the bend by controlling an extent of proximal movement of the rotatable driveshaft within the catheter. The flexible section can be configured to take on an s-shaped curve upon the proximal movement of the rotatable driveshaft within the catheter. The method can further include locking the flexible section in a curved shape by a selected curvature using a handle at a proximal end of the catheter. Moving the rotatable driveshaft distally can include sliding a slider button on a handle of the atherectomy device distally, wherein moving the rotatable driveshaft proximally includes sliding the slider button proximally. The method can further include sliding a slider button on a handle of the atherectomy device proximally to increase a curvature of a flexible section of the elongate body. The method can further include locking the cutter in an active mode where the cutter extends through the cutter window. Moving the rotatable driveshaft proximally can include pressing on and proximally moving a slider button on a handle of the atherectomy device, wherein locking the cutter in the active mode includes releasing the slider button. The method can further include locking the cutter in a passive mode where the cutter is retracted within the cutter window. Moving the rotatable driveshaft distally can include pressing on and distally moving a slider button on a handle of the atherectomy device, wherein locking the cutter in the passive mode includes releasing the slider button. The method can further include rotating the rotatable driveshaft while capturing images outside of the catheter using an imaging sensor coupled to the rotatable driveshaft. The method can further include cutting tissue outside of the catheter by rotating the rotatable driveshaft.

These and other aspects and advantages are described herein.

### BRIEF DESCRIPTION OF THE DRAWINGS

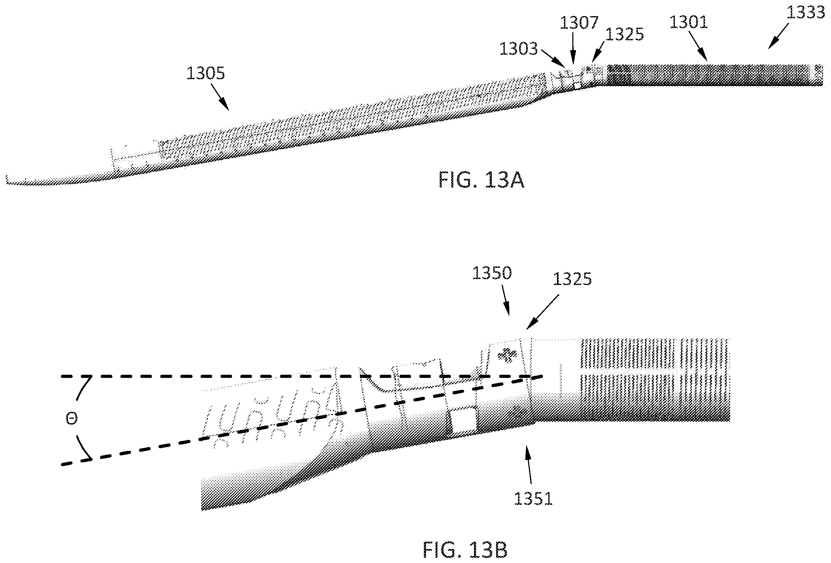

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which FIGS. 13A-14G show embodiments according to the invention, and the rest of the figures are considered useful to understand it.
FIG. 1A shows an atherectomy catheter having a fixed jog.
FIG. 1B shows the atherectomy catheter of FIG. 1A in a vessel.
FIG. 2 shows a drawing of the atherectomy catheter of FIG.1A with relative angles and dimensions.
FIG. 3A shows a variation of a distal end of an atherectomy catheter that includes a user-activated curved portion with stiffening members that cause the catheter to deform to a predetermined curved configuration when activated.
FIG. 3B is a schematic showing the stiffening members of the atherectomy catheter of FIG. 3A.
FIGS. 4A and 4B show another embodiment of an atherectomy catheter with a distal curved portion.
FIG. 5A is a top view of a user-activated curved portion of an atherectomy catheter.
FIGS. 5B and 5C are perspective views of the curved portion of FIG. 5A.
FIG. 5D is a flattened view of the curved portion of FIG. 5A.
FIG. 6A shows an atherectomy catheter including another embodiment of a user-activated curved portion.
FIG. 6B shows the frame of the curved portion of FIG. 6A including annular and longitudinal spines.
FIG. 6C shows a side view of the spine of FIG. 6B.
FIG. 6D shows a cross-sectional view of the spine of FIG. 6B.
FIG. 6E is a flattened view of the spine of FIG. 6B.
FIG. 7A shows a portion of an atherectomy catheter including a fixed jog section and a flexible section.
FIG. 7B shows a flattened view of the curved portion of the catheter of FIG. 7A.
FIG. 8A shows an exemplary flexible nosecone for use with an atherectomy catheter.
FIG. 8B shows a flattened view of a portion of the nosecone of FIG. 8A.
FIG. 9A shows an atherectomy catheter including another embodiment of a user-activated curved portion. The catheter in Figure 9A is in a straightened configuration.
FIG. 9B shows the atherectomy catheter of FIG. 9A in a bent or activated configuration.
FIG. 9C is a bottom close-up view of the curved portion of the catheter of FIG. 9A in a straightened configuration.
FIG. 9D is a top close-up view of the curved portion of the catheter of FIG. 9A in a straightened configuration.
FIG. 9E is a close-up view of the curved portion of the catheter of FIG. 9A in a bent or activated configuration.
FIG. 9F is another close-up view of the curved portion of the catheter of FIG. 9A in a bent or activated configuration.
FIG. 10A shows a close-up partial section view of a catheter having a bushing configured to direct movement of a cutter through a cutting window.
FIGS. 10B, 10C and 10D show close-up partial section view of the catheter of FIG. 10A as the cutter is progressively moved through the cutting window.
FIGS. 11A, 11B and 11C show a side view of a catheter having a flexible section configured to take on a curved shape.
FIG. 12 shows a side view of another catheter having a flexible section configured to take on a curved shape.
FIG. 13A shows a side view of a catheter having a fixed bend.
FIG. 13B shows a close-up view of the fixed bend of the catheter of FIG. 13A.
FIG. 13C shows a close-up partial section view of the catheter of FIG. 13A illustrating a cutter in a passive mode.
FIG. 13D shows a close-up partial section view of the catheter of FIG. 13A illustrating a cutter in an active mode.
FIG. 13E shows a close-up partial section view of the catheter of FIG. 13A illustrating a cutter between the passive mode and the active mode.
FIG. 14A shows a perspective view of an example cutter and bushing for a catheter having a fixed bend.
FIG. 14B shows a section view of the cutter and bushing of FIG. 14A.
FIG. 14C shows a perspective view of a proximal side of the bushing shown in FIG. 14A.
FIG. 14D shows a perspective view of a distal side of the bushing shown in FIG. 14A.
FIG. 14E shows a section view of the bushing shown in FIG. 14A.
FIG. 14F shows another section view of the bushing shown in FIG. 14A.
FIG. 14G shows a section view of the cutter and bushing of FIG. 14A illustrating a detent for retaining the cutter in a passive mode.
FIGS. 15A-15C show section views of a portion of a handle having a slider lock for locking an axial position of the driveshaft with respect to the catheter: FIG. 15A shows the slider lock engaged in a distal position; FIG. 15B shows the slider lock unengaged in the distal position; and FIG. 15C shows the slider lock engaged in a proximal position.

### DETAILED DESCRIPTION

Described herein is an atherectomy catheter having an elongate body with a curved distal portion, a nosecone and a rotatable annular cutter. The curved portion (which can otherwise be called a bent/bendable portion or jog mechanism) can advantageously be used to push the cutter up against the vessel wall to enhance the efficiency of cutting.

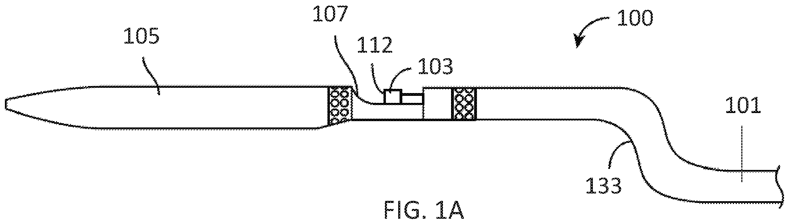

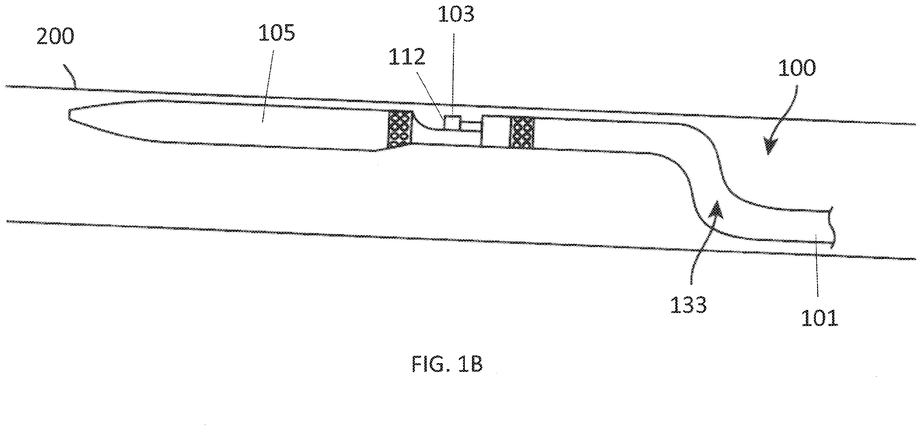

FIGS. 1A and 1B show an exemplary atherectomy catheter 100 having a curved portion along the elongate catheter body. Referring to FIGS. 1A-2, the atherectomy catheter 100 can include a catheter body 101 with a curved portion 133, a rotatable annular cutter 103 at a distal end of the catheter body 101, and a nosecone 105 at a distal end of the catheter body 101. The nosecone 105 can include a cutting window 107 configured to allow the cutter 103 to cut therethrough. The catheter 101 can further include a curved portion 133 in the catheter body 101 to radially push the cutter 103 against the vessel wall.

The curved portion 133 can be a fixed jog (i.e., have a pre-set shape). Further, the curved portion can be curved or bent such that the cutting window 107 is on the radially outermost portion of the curved portion 133 (thereby allowing the cutting window 107 to be urged against a vessel wall in use). In one embodiment, the curved portion 133 can be preformed, for example, by using pre-deflected shaped-set nitinol ribbon segments embedded in the outer shaft. The curved portion 133 can have a shape that advantageously positions the cutter 103 with respect to the vessel wall for cutting. In some cases, the curved portion 133 can have two inflection points 155, 166 of opposite curvature (i.e., one curving up and the other curving down) so as to form an approximate "s" shape. In one embodiment, the s-shape can be configured such that a distal end of the catheter body 101 is offset from, but substantially parallel to, a proximal end of the catheter body 101. In other embodiments, the distal end and proximal ends of the catheter body 101 can be at a slight angle to one another so as to control the angle of cutter engagement with the vessel wall.

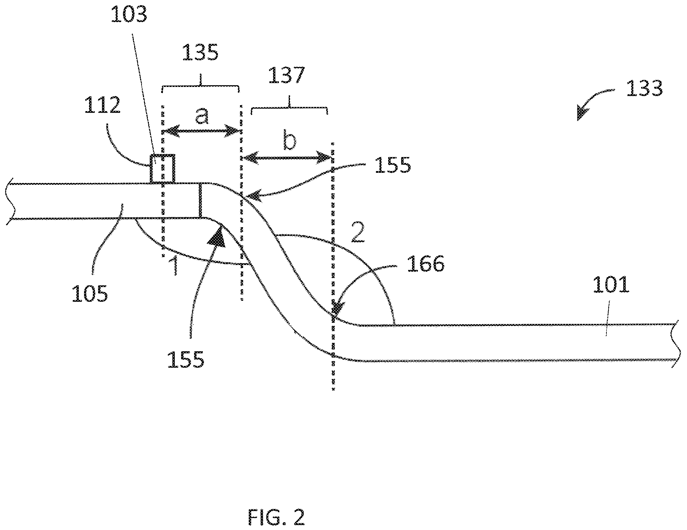

Thus, as shown in FIG. 2, the "s-shape" of the curved portion 133 can include a proximal section 137 have a length *b* that extends from the center of the distal inflection point 155 to the center of the proximal inflection point 166. Further, the curved portion 133 can include a distal section 135 having a length *a* that extends from the cutting edge 112 to the center of the distal inflection point 155. Further, there can be distal angle *1* at the distal end of the "s-shape" and a proximal angle 2 at the proximal end of the "s-shape." These lengths (*a, b*) and angles (1, 2) can be tuned to achieve the desired jog or offset in order to obtain optimum apposition to tissue walls. For example, the length *a* can be shorter than the length *b* to ensure that the cutter is as close to the angle *1* as possible, thereby providing better apposition of the cutter 303. The angles 1 and 2 can be between 120 and 180 degrees, such as between 140 and 160 degrees. In one example, the length *a* is between 5 and 10mm, the length *b* is between 10 and 15mm, the angle *1* is 140 degrees and angle 2 is 160 degrees for a catheter configured to be used in a vessel having a 2.5-4mm diameter.

The curved portion 133 can advantageously radially push the distal end of the catheter against a vessel wall 200, thereby enabling optimized cutting and/or imaging of the vessel as shown in FIG. 1B.

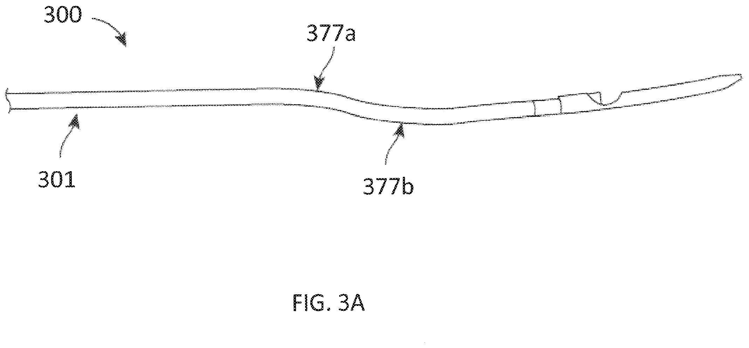

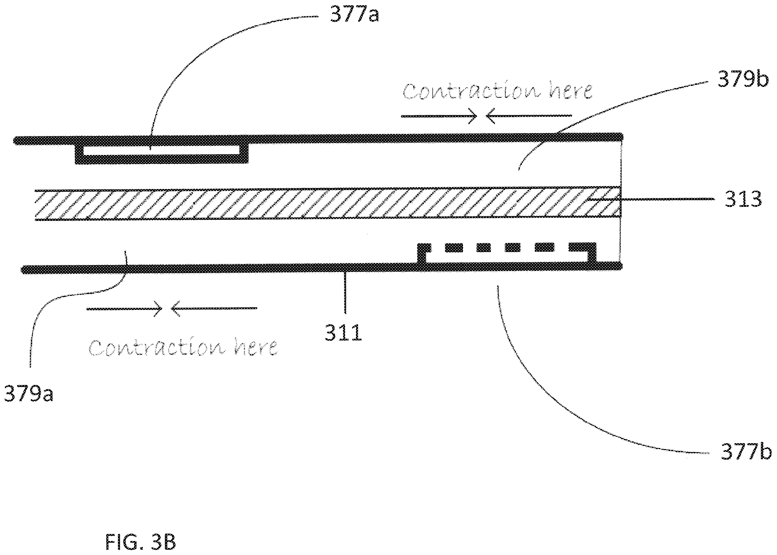

FIGS. 3A-3B show another embodiment of an exemplary catheter 300 that includes a curved portion 333 in the catheter body that urges the atherectomy cutter against the vessel wall. The curved portion 333 can have similar dimensions and features as curved portion 133. In contrast to the fixed jog curved portion 133 of catheter 100, however, the curved portion 333 can be a user-activated jog. Thus, referring to FIGS. 3A and 3B, the catheter 300, can be deflected into a curved portion 333 by tensile and compressive interaction between an inner shaft 313 (which can be a drive shaft for a cutter) and outer shaft 311 that are fixed together at the distal end but free to move relative to one another at the proximal end. The outer shaft 311 can include stiffening members 377a,b, such as nitinol or stainless steel, stiffening members, configured to bias the deflection to a set shape. As shown in Figure 3B, there can be two stiffening members 377a, 377b that can be axially aligned with the outer shaft 311 and axially and radially offset from one another. As a result, when compression is applied on the outer shaft 311 (such as by pulling on the inner shaft 313 or a separate pullwire or shaft), the portions 379a,b of the outer shaft opposite to the stiffening members 377a,b will contract. The contraction of the two portions 379a, 379b will result in an s-shape similar to the catheter 100 shown in FIG. 1. As a result, the catheter will deflect into jog or s-shaped configuration where the distal end of the shaft is offset and parallel to the main shaft body. It is to be understood that other numbers and arrangements of stiffening members are possible, as are other resulting jog shapes.

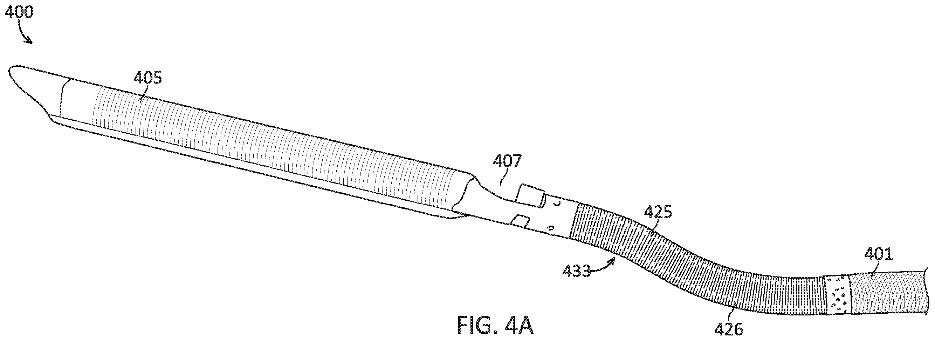

Another embodiment of an atherectomy catheter 400 including a user-activated curved portion 433 is shown in FIGS. 4A-4B. The atherectomy catheter 400 includes an elongate body 401, a nosecone 405 attached thereto, and a cutting window 407 configured to expose an annular cutter 411 therethrough. Moreover, the catheter 400 includes a curved portion 433. The curved portion 433 includes curved sections 425, 426 of opposite curvatures (i.e., one curving up and the other curving down) so as to form an approximate s-shape. In one embodiment, the s-shape can be configured such that the distal end of the catheter body 401 and/or the nosecone 405 is offset from, but substantially parallel to, a proximal end of the catheter body 401. In another embodiment, the distal end of the elongate body 401 and/or the nosecone 405 forms an angle relative to a proximal end of the catheter body 401.

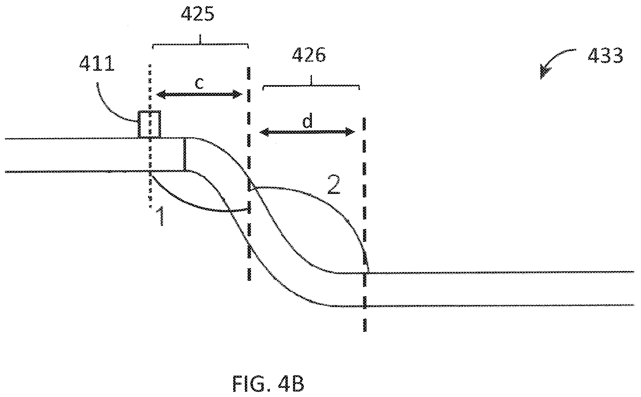

Thus, as shown in FIG. 4B, the "s-shape" of the jog 433 can have a proximal curved section 426 and a distal curved section 425 having a length c. Further, there can be distal angle *1* at the distal end of the "s-shape" and a proximal angle 2 at the proximal end of the "s-shape." The lengths (c, *d*) and angles (*1, 2*) of the jog 433 can be tuned to achieve the desired jog or offset in order to obtain optimum apposition to tissue walls. For example, the angles *1* and *2* can be between 120 and 175 degrees, such as between 140 and 160 degrees. Further, in some embodiments, the length d of the proximal section 426 is greater than the length c of the distal section 425. In one example, the length *c* is 5mm, the length *d* is 8mm, and the angles *1* and *2* are 150 degrees for a catheter configured to be used in a vessel having a 2.5-4mm diameter. The curved portion 433 can be a configured to adopt the s-shape during use of the catheter, as described above with respect to curved portion 333.

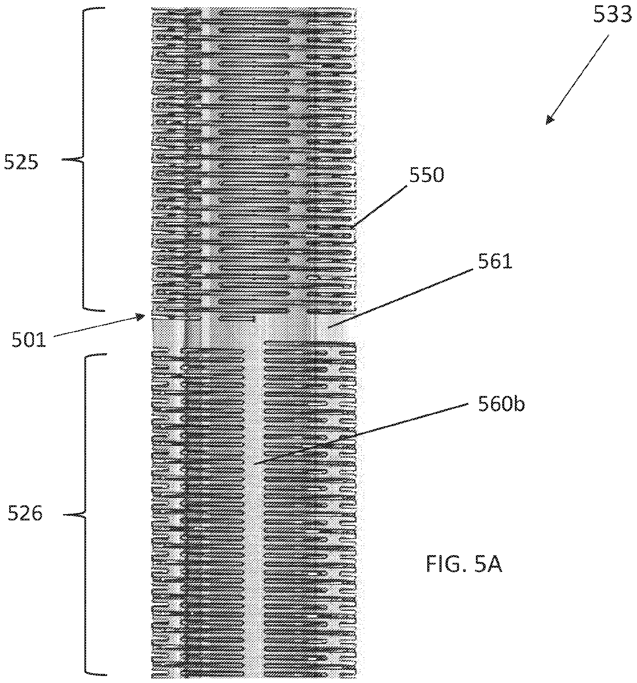

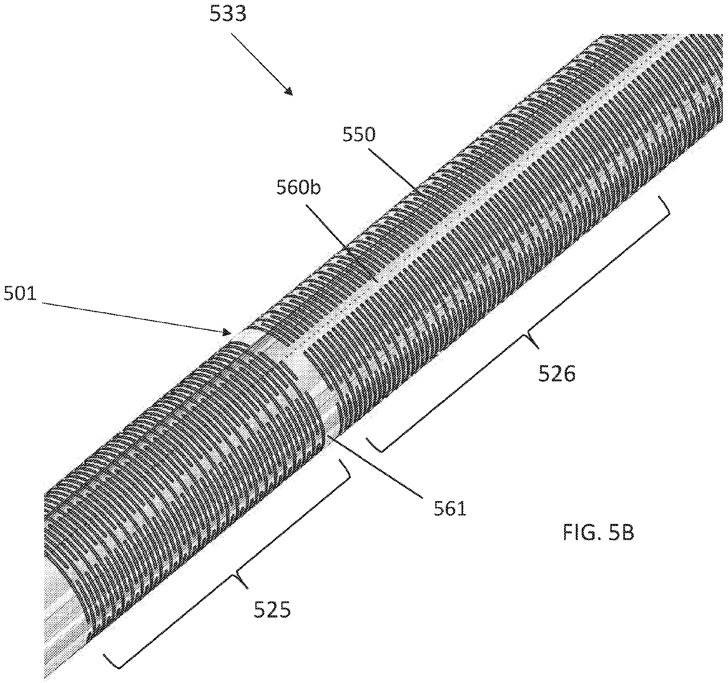

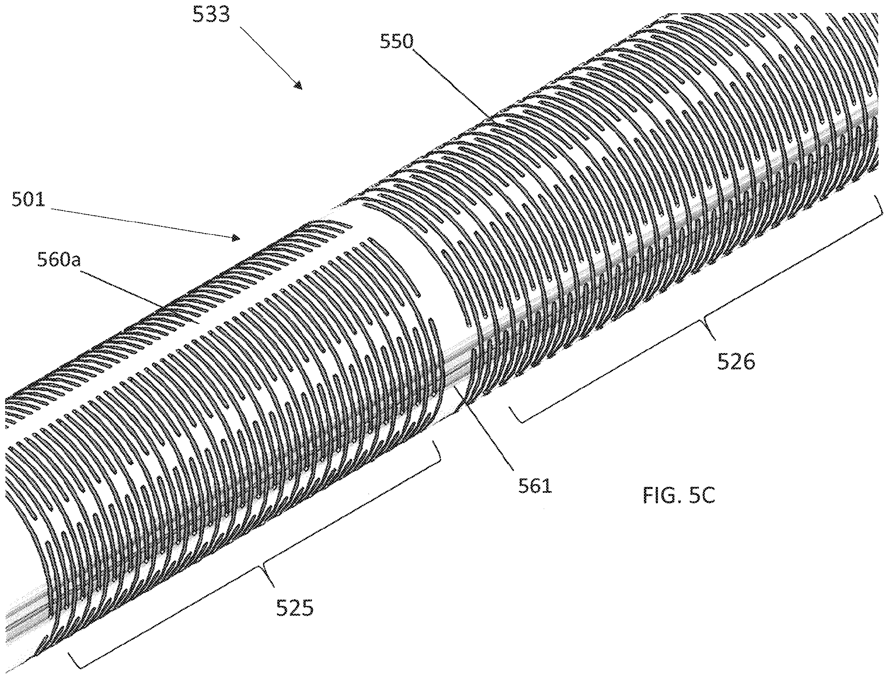

An exemplary user-activated curved portion 533 (e.g., for use as curved portion 433) is shown in FIGS. 5A-5D. The curved portion 533 can include a frame (e.g., made of Nitinol or stainless steel) including a series of circumferential slits 550 (e.g., laser cuts) that are patterned along the circumference of the elongate body 501 within the curved sections 525, 526. The frame of the curved sections 525, 526 can also include a longitudinal spine 560a,b (also referred to herein as a backbone) extending therethrough. The longitudinal spines 560a,b can be positioned approximately 180 degrees away from one another (i.e., on opposites sides of the elongate body 501) and extend substantially parallel to the longitudinal central axis of the elongate body 501. The frame can further include a circumferential spine 561 separating the two curved sections 525, 526. Each spine 560a,b and 561 is formed of a substantially solid piece of material that does not include slits therein. In use, as the circumferential slits 550 compress and/or overlap with one another during bending, the longitudinal spines 560a,b form the backbone of the curved sections 525, 526. Further, in some embodiments, the frame can be laminated with a layer thereover and/or under, such as a thin polymer layer, such as Tecothane. In other embodiments, the frame is not laminated to provide for greater flexibility.

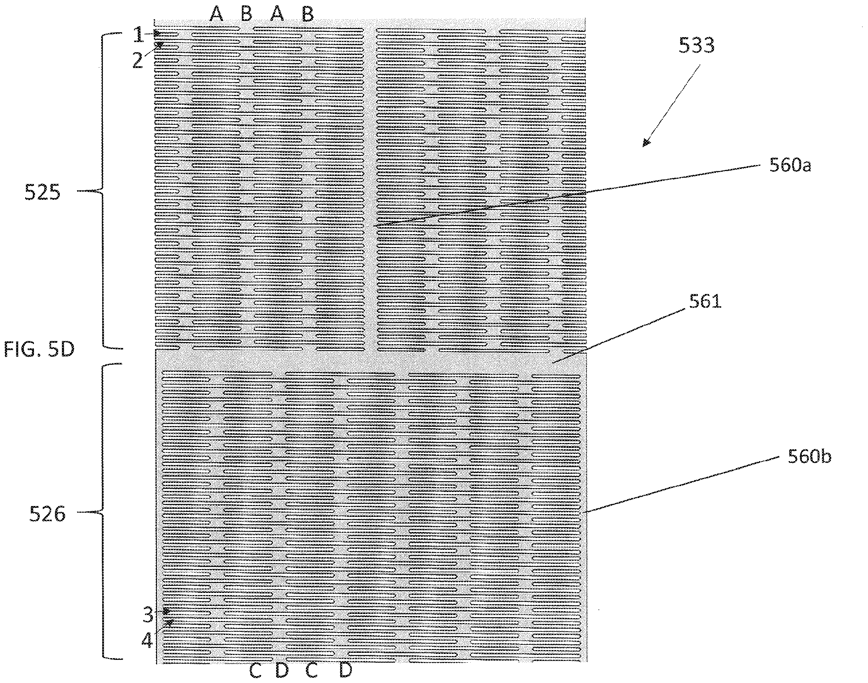

Referring to FIG. 5D, the slits 550 can be arranged in a pattern that is configured to provide flexibility while maintaining structural integrity of the elongate body. Thus, the majority of the slits 550 can have the same length, but be offset from one another. For example, the slits in distal section 525 can be arranged in rows (1,2) and columns (A, B). Each slit 550 (except the shorter slits bordering the spine 560a) can have a length equivalent to the width of columns A + B + A. Further, the slits can be offset from one another by a distance of A + B. Thus, each column A can include slits from every row 1,2 while column B can include alternating slits (from either row 1 or 2). Column B thus provides structural integrity to the slitted portion of the device. The slits in section 526 can be similarly arranged, but can be offset such that each column C (with slits from every row 3,4) is aligned with the central axis of each column D (with slits from row 3 or 4). The offset helps provide stability to the catheter as it bends.

In some examples, pushing or pulling on a shaft of the catheter, such as the cutter drive shaft, a pullshaft, or a pullwire can activate the curved portion 533. That is, as the shaft is pulled back proximally, it can place compression on the outer elongate body 501, causing the slits 550 to compress and/or move over one another while the spines 560a,b maintain their length. The resulting s-shape (see Figure 4B) allows the cutter (just distal to spine 460a) to be pushed up against the vessel wall.

The slits 550 shown in FIGS. 5A-5D are of a repeated, symmetrical pattern. However, the pattern need not be symmetrical. In some embodiments, the slits can all have the same length. In other embodiments, some of the slits are longer than others. In one embodiment, the slits are .0016" wide and .0575" long with a .0035" offset from the next row of slits.

Areas of the catheter body having a greater degree of slits will be more flexible than those having lesser degrees of slits. In one embodiment, the slits can extend all the way through the elongate catheter. In other instances, some of the slits may be deeper or shallower than others which also affects the flexibility of the corresponding region. In some variations of the curved portion, a range of deflection between the flexible segments may be achieved. This may be accomplished through different geometric patterns of slits, different spacing of the slits, frequency of the slits, size of the slits, and so forth. In some instances, the degree of stiffness may be adjusted by adding additional spines of various lengths in certain areas or adjusting the width of the spines.

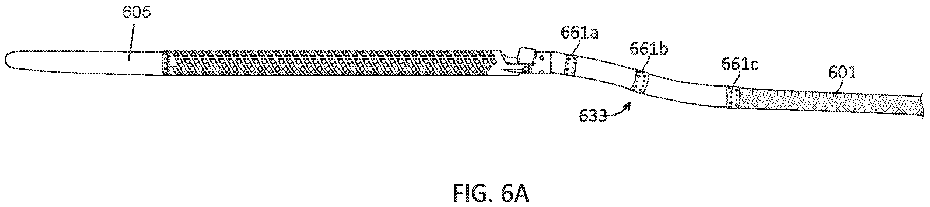

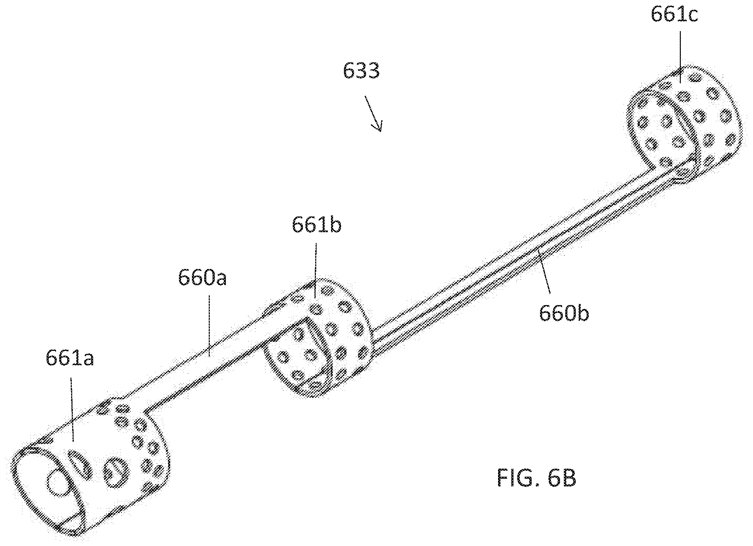

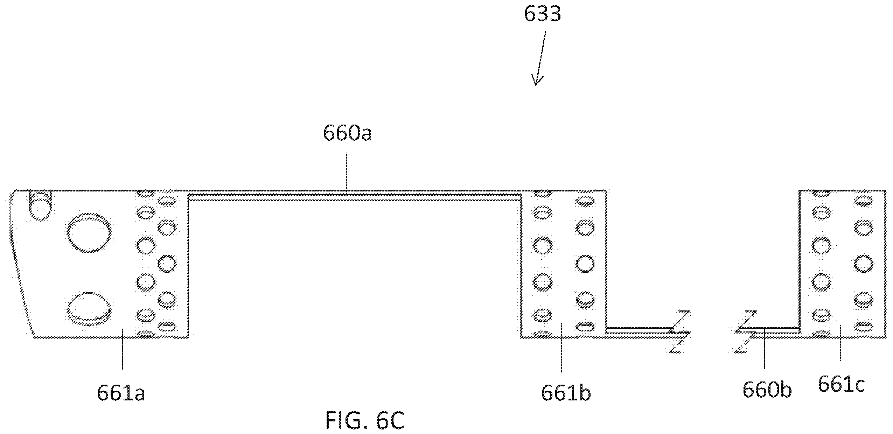

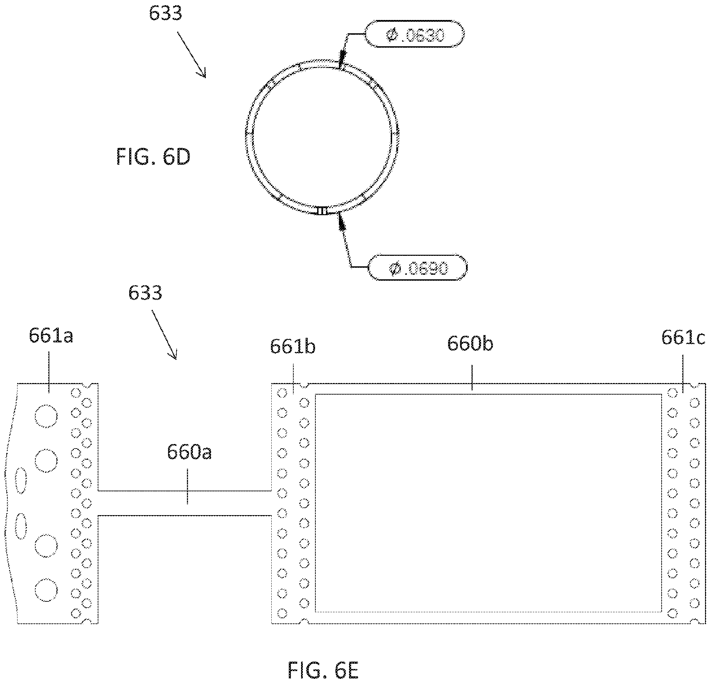

Referring to FIGS. 6A-6E, another exemplary curved portion 633 (e.g., for use as curved portion 433) is shown. The curved portion 633 includes a frame having three annular ring spines 661a,b,c connected together by two longitudinal spines 660a,b. The longitudinal spines 661a,b,c can be approximately 180° away from one another. In some embodiments, the distal ring 661a,bc can be beveled at the distal end, as shown in FIG. 6C, to allow for dropping or pivoting of the nosecone 605. Further, the space between the annular ring spines 661a,b,c and the longitudinal spines 660a,b can be open or cut-away (i.e., not include the frame material). In some embodiments, the frame can be laminated to the elongate body 601 with one or more thin polymer layers, such as Tecothane. The ring 661a,b,c can include holes therein for soldering or laminating the mechanism 633 to the elongate body of the catheter. In other embodiments, the frame can remain unlaminated to provide for greater flexibility. When compression is placed upon the mechanism 633, the mechanism 633 can bend away from each of the spines 660, forming an s-shape. For example, compression can be placed on the mechanism 633 by pulling the driveshaft is pulled proximally.

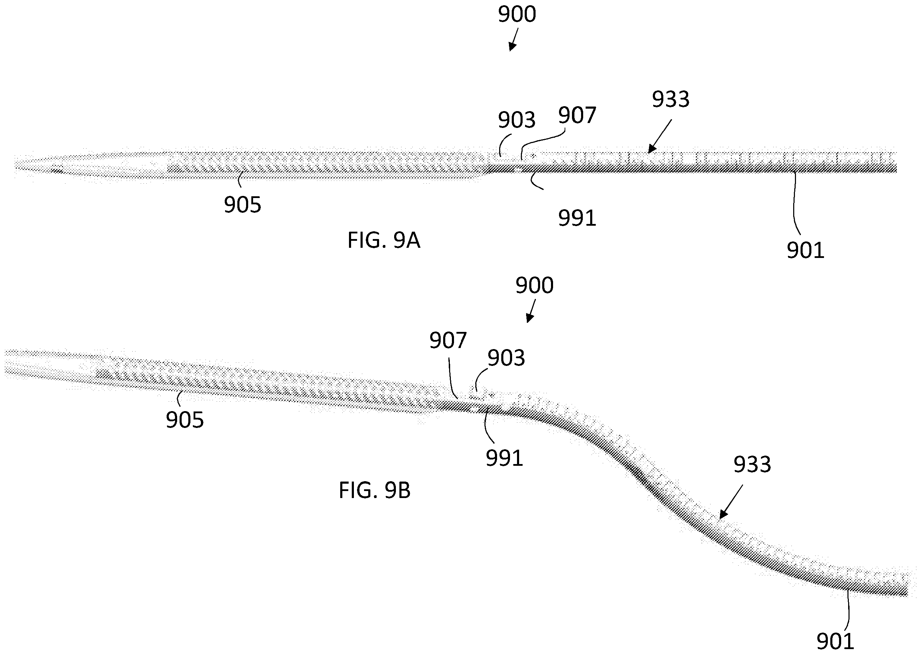

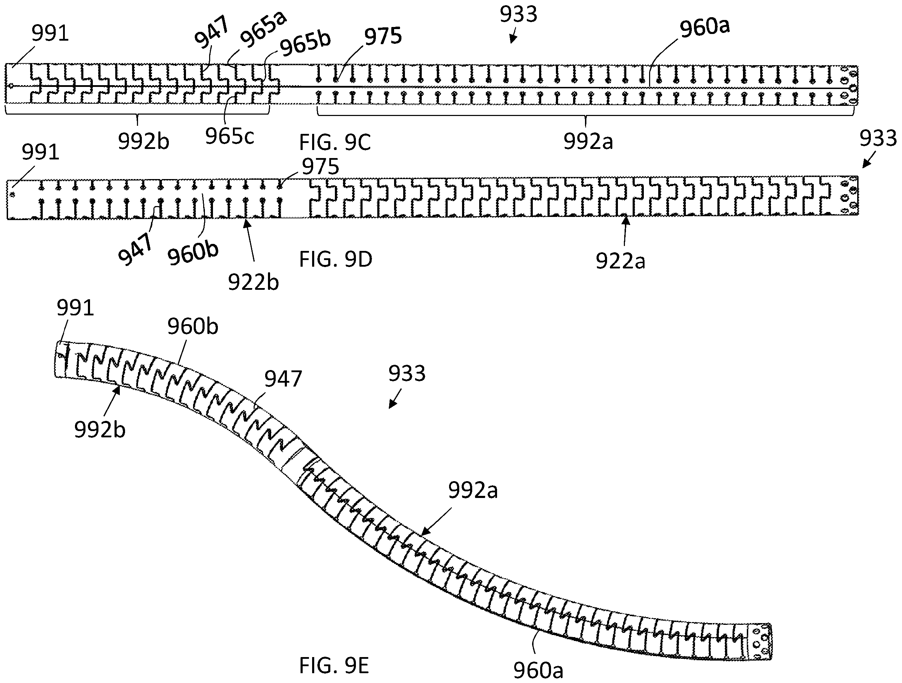

Referring to FIGS. 9A-9F, another exemplary atherectomy catheter 900 with a curved portion 933 is shown. The atherectomy catheter 900, like atherectomy catheter 100, can include a catheter body 901 with curved portion 933, a rotatable annular cutter 903 at a distal end of the catheter body 901, and a nosecone 905 at a distal end of the catheter body 901. The catheter body 901 and/or nosecone 905 can further include a cutting window 907 configured to allow the cutter 903 to cut therethrough. The catheter 900 can further include a driveshaft (not shown) attached to the cutter 903 and configured to rotate the cutter 903 when activated. In this embodiment, the nosecone 905 is not hinged relative to the elongate body 901. Rather, the bushing 991 of the elongate body 901 attaches directly to the proximal end of the nosecone 905. Not having a hinge can advantageously prevent the nosecone 905 from getting caught within the vessel. In this embodiment, proximal or distal movement of the cutter 903 and driveshaft can activate the curved portion 933 to push the cutter 903 radially against the vessel well (e.g., outside the cutting window 907).

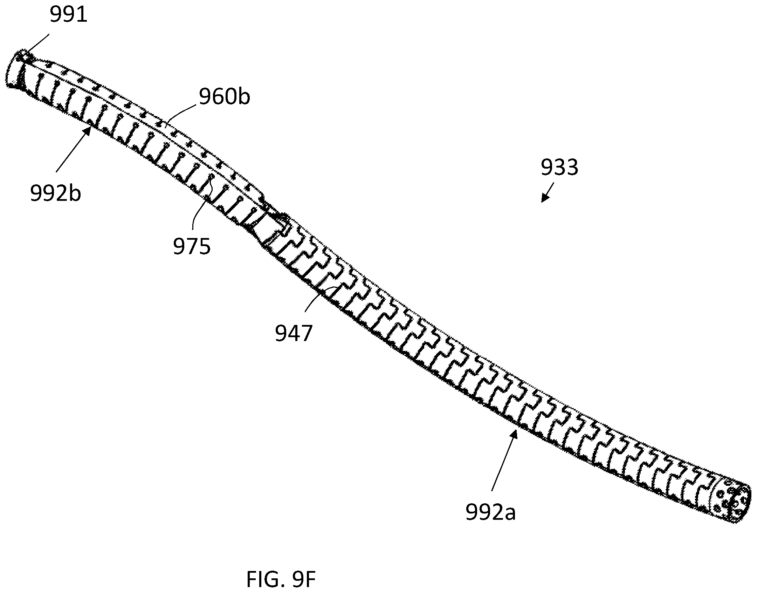

The curved portion 933 includes a tubular frame having a proximal section 992a and a distal section 992b. Each section 992a, 992b includes a longitudinal spine 960a,b. The longitudinal spines 960a,b are positioned approximately 180 degrees away from one another. Circumferential cuts 947 (e.g., laser cuts) having a jigsaw pattern that define tongue elements 965a,b,c (see FIG. 9C) can be positioned opposite each spine 960a,b. In some embodiments, the jigsaw pattern includes holes 975 where the cuts 947 terminate at the spines 960a,b. When compression is placed upon the mechanism 933, the mechanism 933 can bend into an s-shape with the proximal section 992a bending in a first direction and the distal section 992b bending in a second opposite direction. As shown in Figure 9F, during such bending, the spines 933c do not compress and thereby forming the outer diameter of each respective curve and the cuts 947 compress together to form the inner diameter of each respective curve.

In some embodiments, the tongue elements 965a,b,c can have a tapered structure configured to dictate the amount of deflection of the curved portion 933 in both directions. For example, the tongue elements 965a,b,c can lock with respect to one another in the curved position, thereby keeping the curved portion 933 aligned and resistant to twisting when under torsion when in the curved or deflected position. This can also prevent the curved portion 933 from over-bending.

In some embodiments, the proximal section 992a can be longer than the distal section 992b. For example, the proximal section 992a can form 60-90%, such as 65%-70% of the length of the curved portion 933 while the distal section 992b can form 10%-40%, such as 30%-35% of the length of the curved portion 933. Having a longer distal section 992b than proximal section 992a can advantageously help ensure that the cutter 903 is forced against the vessel well during use without tipping back down towards the center of the vessel.

The curved portion 933 can be coupled to the outer shaft of the atherectomy catheter using any technique, such as welding, adhesive, fastener(s), or a combination thereof (e.g., via holes 907).

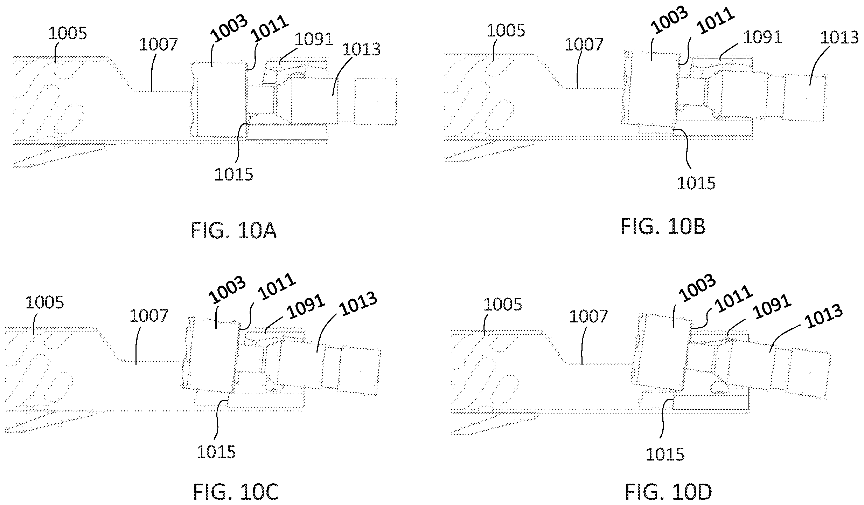

Referring to Figures 10A-10D, in any of the embodiments described herein, the cutter 1003 can include a proximal ledge 1011 configured to interact with the bushing 1091 on the curved portion 1033 when the driveshaft 1013 is pulled proximally. The curved portion 1033 can correspond to a flexible section of the catheter. In embodiments wherein the nosecone 1005 is not hinged, the interaction between the proximal ledge 1011 and the bushing 1091 can cause the cutter 1033 to move through the window 1007. For example, the distal edge 1015 of the bushing 1091 can be angled such that, as the drive shaft 1013 is pulled proximally, the ledge 1011 slides along the sloped distal edge 1015 (e.g., sloped with respect to an axis perpendicular to the longitudinal axis of the nosecone 1005) to move the cutter 1003 out of the cutter window 1007 (see the movement from Figures 10A-10D). That is, interaction between the ledge 1011 and the edge 1015 can cause at least a portion of the cutter to extend through (e.g., pop through) the window 1007 and tilt such that the cutter 1003 is at an angle relative to the nosecone 1005 and the elongate body (e.g., 901 in FIGS. 9A and 9B). In other words, movement of cutter 1033 proximally relative to the nosecone can cause at least a portion of the cutter to extend through the window and cause a longitudinal axis of the cutter 1033 to become non-parallel to a longitudinal axes of the nosecone 1005 and the elongate body. In some embodiments, the longitudinal axis of the cutter 1033 is at an angle ranging from about 1 degree and 30 degrees (e.g., 1°-30°, 5°-30°, 20°-30°, 1°-20°, or 10°-20°) relative to the longitudinal axes of the nosecone 1005 and elongate body when the cutter 1003 is fully deployed.

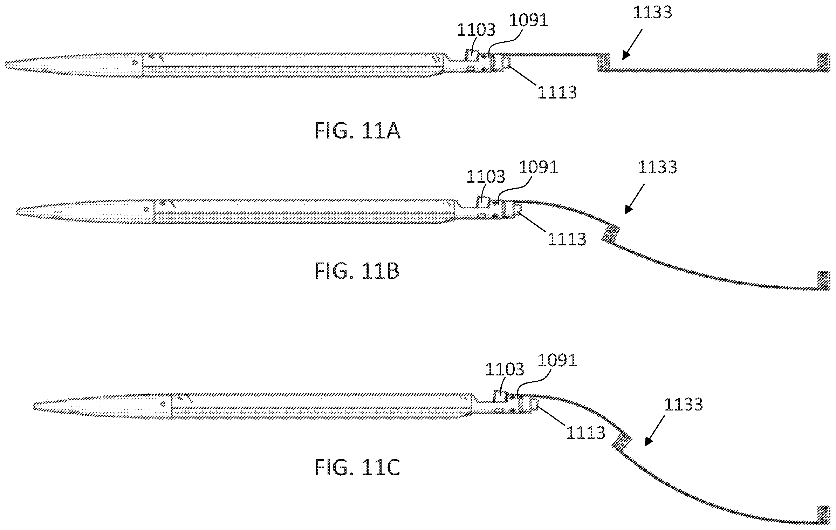

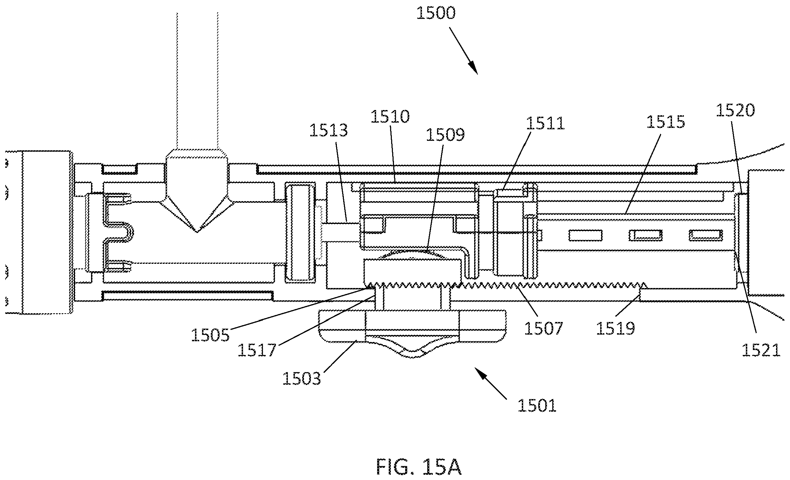

In some embodiments, the interaction between the proximal ledge and the bushing can additionally or alternatively cause the curved portion (also referred to as a flexible section) to assume its s-shape. For example, referring to Figures 11A-11C, pulling the driveshaft 1113 (shown cut off for clarity) proximally can cause the cutter 1103 to engage with the bushing 1191 to place compression on the curved portion 1033 and force the curved portion 1133 to bend (i.e., away from each of the longitudinal spines 1160a,b). In some embodiments, the amount of curvature can be incrementally and/or continuously adjustable by placing varying amounts of compression on the curved portion 1133 via the driveshaft. Likewise, pushing the driveshaft 1113 distally can straighten the curved 1133. A locking mechanism, such as a mechanism on the handle, can fix the curved portion 1133 at the desired amount of curvature. An example of a handle with locking mechanism is described below with reference to FIGS. 15A-15C.

In some embodiments, the cutter 1003 is pulled proximally by a first extent and/or at a first time to extend a portion of cutter 1003 through the window 1007 and tilt relative to the nosecone and elongate body (e.g., as shown in FIGS. 10A-10D), and pulled proximally by a second and/or at a second time to cause the flexible portion to bend and assume an s-shape to varying degrees depending on the amount of force applied to the driveshaft in the proximal direction (e.g., as shown in FIGS. 11A-11C).

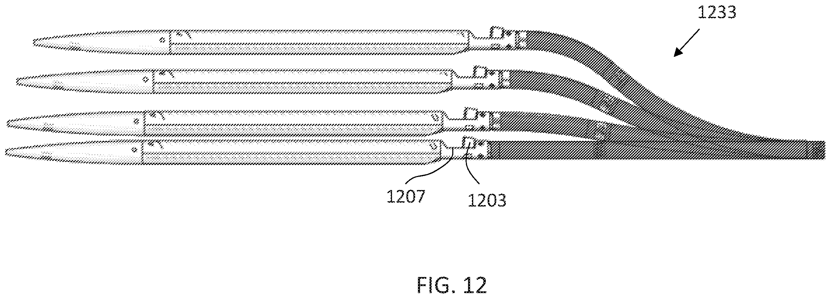

Referring to Figure 12, in some embodiments, pulling the cutter 1203 proximally (e.g., via the driveshaft) can first cause the cutter 1203 to pop out of the cutter window 1207. Further proximal pulling on the cutter 1203 can then cause the curved portion 1233 to assume the desired s-shape (e.g., in a continuously adjustable manner). Having the cutter 1203 pop out of the window first can advantageously ensure that the cutter 1203 can be fully extended regardless of the assumed curvature. Pushing the cutter distally can cause the curved portion 1233 to straighten to a desired extent. Further distal pushing on the cutter 1203 can cause the cutter 1203 to retract within the window 1207.

Referring to FIGS. 13A and 13B, the nosecone 1305 can be fixedly coupled to or integrally formed with the elongate body 1301 at a fixed angle Θ relative to the elongate body. This fixed angle can form a fixed bend 1325 (also referred to as a fixed curve) in the catheter between the nosecone 1305 and the elongate body 1301. A cutting window 1307 can provide access to the lumen of the catheter where the cutter 1303 is housed. The cutting window 1307 can be located adjacent to or at the fixed bend 1325. In some embodiments, the cutting window 1307 is distally located along the catheter with respect to the bend. The cutter 1303 can be configured to extend through the cutting window 1307 upon translation of the cutter 1303 and driveshaft relative the catheter (i.e., nosecone and elongate body). For example, the driveshaft and cutter 1303 can be pulled to move the cutter 1303 proximally and extend the cutter 1303 though the cutting window. Likewise, the driveshaft and cutter 1303 can be pushed to move the cutter 1303 distally and retract the cutter 1303 into the catheter housing.

The cutting window 1307 can be on a convex side 1350 of the catheter formed by the bend (e.g., as opposed to a concave side 1351 of the catheter formed by the bend). This configuration can provide the rotating cutter 1303 better access to material outside of the catheter for cutting. The angle Θ of the bend 1325 can vary. In some embodiments, the angle Θ ranges from about 1 degree and 30 degrees (e.g., 1°-30°, 5°-30°, 20°-30°, 1°-20°, or 10°-20°). Thus, in some embodiments, the angle of the bend 1325 at the convex side 1350 of the catheter may range from about 181° and 210° (e.g., 181°-210°, 186°-210°, 200°-210°, 186°-200°, or 190°-200°).

The elongate body 1301 can include a flexible section 1333 consistent with the flexible section as described above with reference to FIGS. 11A-11C and 12. For example, the flexible section 1333 can be incrementally and/or continuously adjustable to take on an s-shape to varying degrees depending on an amount of proximal movement of driveshaft relative to the nosecone 1305 and elongate body 1301. The flexible section 1333 can have a greater flexibility than the nosecone 1305, the bend 1325, and in some cases a remainder of the catheter.

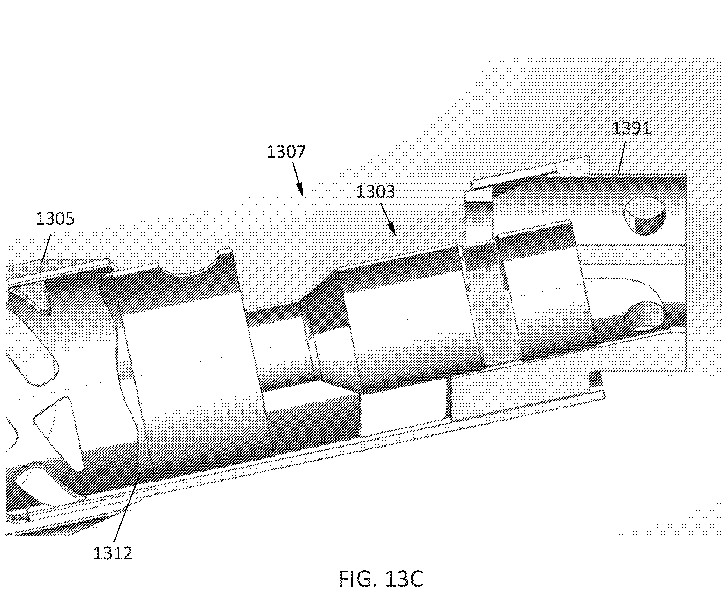

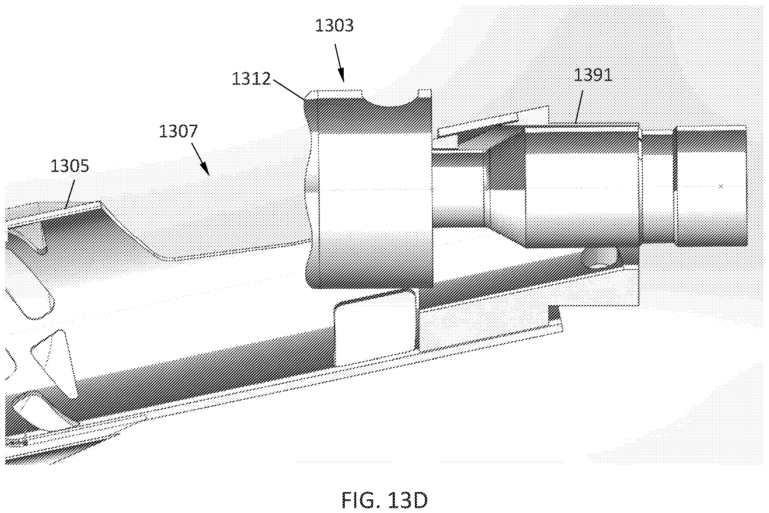

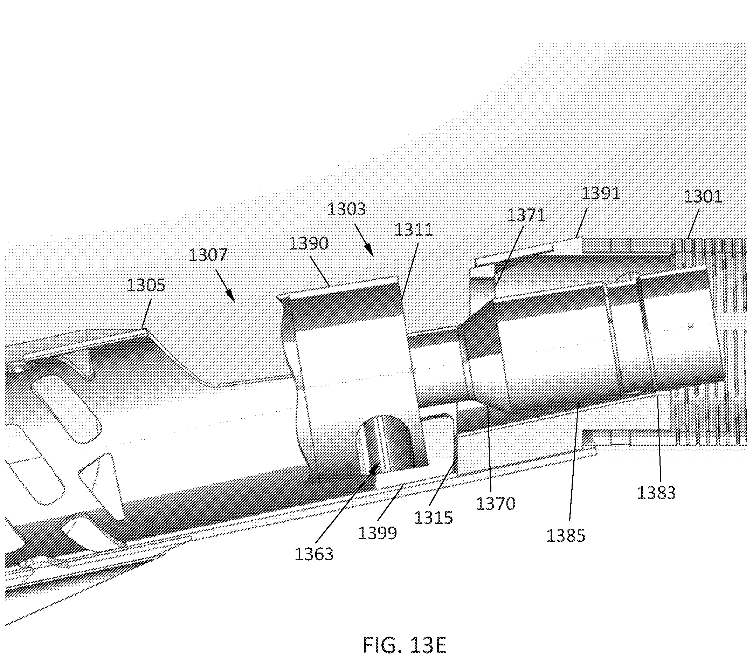

FIGS. 13C-13E show close-up partial section views of the bend 1325. The cutter 1303 can be configured to transition between a passive mode, such as shown in FIG. 13C, and an active mode, such as shown in FIG. 13D. FIG. 13E shows the cutter 1303 between the passive mode (FIG. 13C) and active mode (FIG. 13D).

Referring to FIG. 13C, when the cutter 1303 is in passive mode, the cutting edge 1312 of the cutter 1303 can be distally located with respect to the window 1307 and housed within the nosecone 1305 such that the cutting edge 1312 of the cutter 1303 is fully protected and does not extend through the cutting window 1307. In the passive mode, the cutting edge 1312 may be prevented from cutting material outside of the catheter, thereby preventing the cutting edge 1312 from cutting tissue, for example, a vessel wall. The cutter 1303 may be placed in the passive mode, for example, as the catheter is being maneuvered through the vessel to arrive at a target location within the vessel (e.g., to remove material such as plaque) and/or being withdrawn from the vessel (e.g., after removal of the material from the vessel). In the passive mode, a longitudinal axis (e.g., axis of rotation) of the cutter 1303 can be substantially parallel to a longitudinal axis of the nosecone 1305. In the passive mode, the cutter 1303 can be pushed distally toward the nosecone 1305 to, for example, pack material (e.g., plaque) into the nosecone 1305.

Referring to FIG. 13D, when the cutter 1303 is in the active mode, the cutting edge 1312 of the cutter 1303 can extend through the cutting window 1307. The cutter 1303 (e.g., the cutting edge 1312 of the cutter 1303) can extend beyond the outer walls on the convex side of the catheter so that the cutter 1303 can efficiently access material outside of the catheter for cutting. For example, at least a portion of the cutter 1303 (e.g., at least a portion of the cutting edge 1312) can correspond to the most prominent feature or point along the convex side of the bend when the cutter 1303 is extended through the cutting window 1307 (e.g., fully extended in the active mode). In the active mode, a longitudinal axis (e.g., axis of rotation) of the cutter 1303 can be substantially parallel to a longitudinal axis of the elongate body (e.g., 1301, FIG. 13A). Since the cutter 1303 can be parallel to the elongate body, the cutter 1303 can be at the angle Θ (FIG. 13B) relative to the nosecone 1305 when in the active mode.

FIG. 13E shows the cutter 1303 between the passive mode and the active mode. The cutter 1303 and driveshaft can be moved proximally (e.g., pulled away from the nosecone 1305) to transition the cutter 1303 from the passive mode to the active mode. Likewise, the cutter 1303 and driveshaft can be moved distally (e.g., pushed toward the nosecone 1305) to transition the cutter 1303 from the active mode to the passive mode. During transition between the passive and active modes, the cutter 1303 can be configured to interact with inner surfaces within the catheter to adjust the position and orientation of the cutter 1303 relative to the nosecone 1305 and the elongate body 1301.

During transition from the passive mode to the active mode, the cutter 1303 (e.g., via the driveshaft) is pulled proximally to cause a proximal ledge 1311 (also referred to as a proximal face) of a head 1390 of the cutter 1303 to slide along a distal edge 1315 of a bushing 1391. This interaction causes the cutter 1303 to move radially outward with respect to a central axis of the elongate body 1301 and extend through the cutting window 1307 (e.g., pop out of the window). This interaction also causes the cutter 1303 to tilt such that a longitudinal axis of the cutter 1303 aligns with (e.g., becomes substantially parallel to) a longitudinal axis of the elongate body 1301.

During transition from the active mode to the passive mode, the cutter 1303 (e.g., via the driveshaft) is pushed distally to cause a slanted surface 1370 along the shaft 1385 of the cutter 1303 to slide along an internal edge 1371 of the bushing 1391 to cause the cutter 1303 to move radially inward with respect to a central axis of the elongate body 1301 and retract into the catheter. When the cutter 1303 is moved radially inward, the shaft 1385 of the cutter 1303 contacts an internal surface 1383 of the bushing 1391, causing the cutter 1303 to tilt such that the longitudinal axis of the cutter 1303 aligns with (e.g., becomes substantially parallel to) a longitudinal axis of the nosecone 1305.

The transitions between the passive and active modes can be continuous, where the cutter 1303 progressively translates, tilts and moves radially. The cutter 1303 and drive shaft can freely rotate while in the passive mode and the active mode. In some cases, the cutter 1303 can also freely rotate while transitioning between the passive mode and the active mode.

The cutter 1303 can be locked in either the passive or active modes using a locking mechanism of the handle. An example of a locking mechanism is described below with reference to FIGS. 15A-15C.

Any of the catheters described herein may include imaging capabilities such as described in International Application Nos. PCT/US2017/040431 and PCT/US2019/028415, each of which is cited for reference. For example, the cutter 1303 can include a cavity 1363 for an imaging sensor within the catheter to send and/or receive image data as part of an imaging system. The cutter 1303 may be configured to collect imaging data while in the passive mode, the active mode and/or while transitioning between the active and passive modes. In some embodiments, the catheter includes one or more openings 1399 that act as an additional window and/or as a location marker(s) for the imaging sensor.

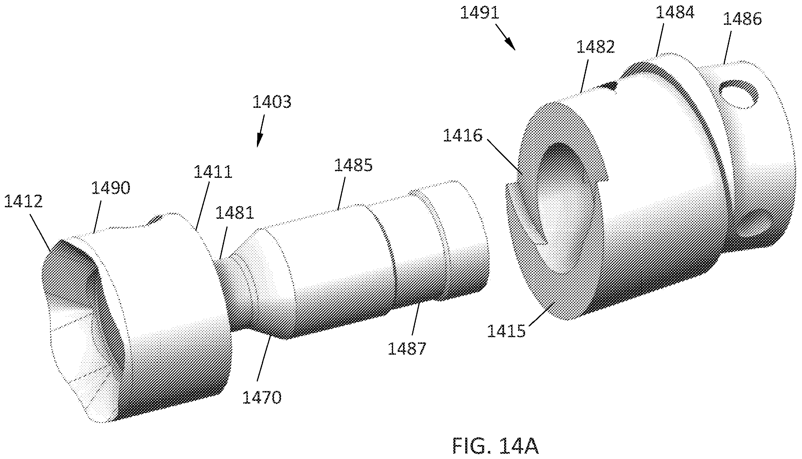

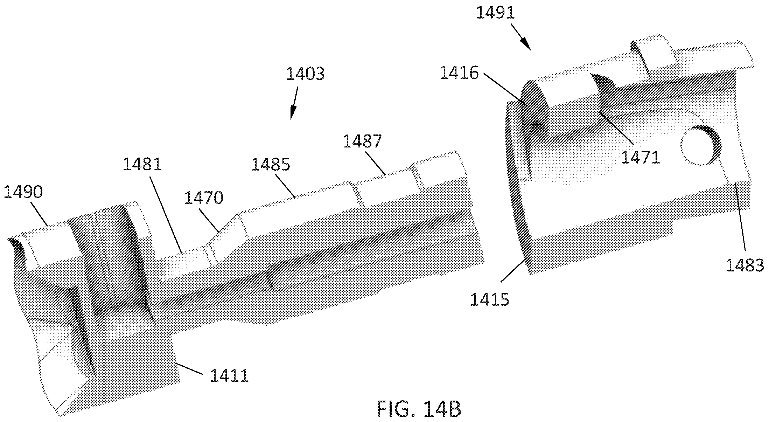

FIGS. 14A and 14B show perspective and section views of an example cutter 1403 and bushing 1491. The cutter 1403 can include a head 1490 at a distal end, a neck 1481, and a cylindrical shaft 1485 at a proximal end. The head 1490 can include an annular cutting edge 1412, which may scalloped in some embodiments. The neck 1481 can have a smaller diameter than the head 1490 and the proximal shaft 1485 to provide a clearance for the cutter 1403 when rotating in the active mode. In some embodiments, the shaft 1485 can include an annular groove 1487 that cooperates with the bushing 1491 to function as a detent (described in detail below). The bushing 1491 can be fixedly coupled to and be positioned between the distal nosecone (e.g., 1305, FIG. 13A) to the proximal elongate body (e.g., 1301, FIG. 13A). In some cases, the bushing 1491 is welded to the nosecone and/or the elongate body. The bushing 1491 can include a first portion 1482 that is substantially parallel the nosecone, and a second portion 1486 that is substantially parallel the elongate body. An intervening portion 1484 can be between the first portion 1482 and the second portion 1486.

As described above, features of the bushing 1491 interact with the cutter 1403 to control movement of the cutter 1403 between active and passive modes. When the cutter 1403 is pulled proximally (e.g., from the passive mode to the active mode), the proximal ledge 1411 (also referred to as a proximal face) of the head 1490 of the cutter 1403 can be configured to slide along a distal edge 1415 of the bushing 1491. This interaction causes the cutter 1403 to move radially outward and extend through the cutting window. This cutter 1403 becomes positioned within a notch 1416 (also referred to as a seal or indentation) of the distal face of the bushing 1491, which provides a space for the proximal ledge 1411 of the cutter 1403 to rotate in the active mode. As shown in FIG. 14A, the distal edge 1415 may form a crescent-shaped step in accordance with the cylindrical head 1490 of the cutter 1403.

Referring to FIG. 14B, the bushing 1491 can include an internal edge 1471 that is configured to slide along an angled surface 1470 the cutter 1403 when the cutter is pushed distally (e.g., from the active mode into the passive mode), causing the cutter 1403 move radially inward. Additionally, an internal surface 1483 of the bushing 1491 contacts the shaft 1485 of the cutter 1403 to cause the cutter 1403 to tilt and move in alignment with and retract within the nosecone, as described above.

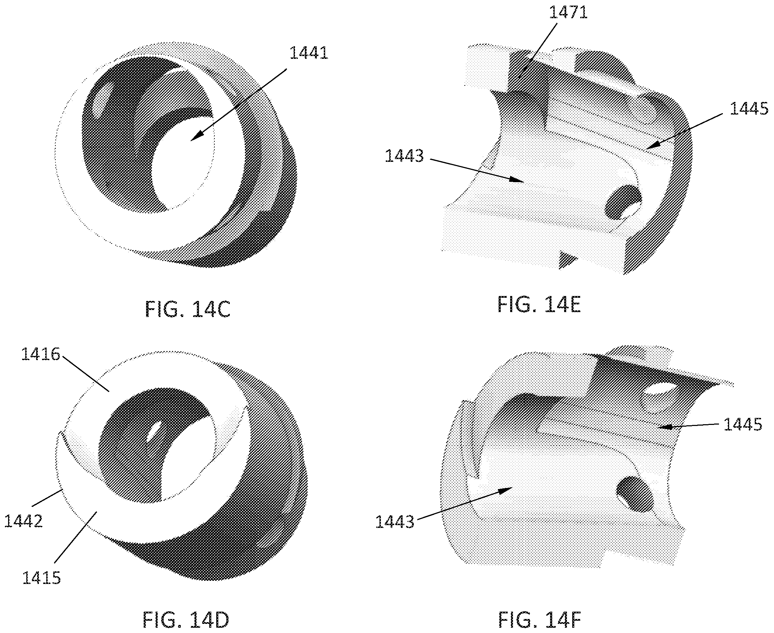

FIGS. 14C-14F shows alternate views of the bushing 1491, illustrating a proximal side (FIG. 14C), a distal side (FIG. 14D), and section views (FIGS. 14E and 14F) of the bushing. A proximal opening 1441 of the bushing can have an oblong shape to provide clearance for the shaft of the cutter when transitioning between the active and passive modes. The internal surfaces of the bushing can form a first channel 1443 for the cutter while in the passive mode and a second channel 1445 for the cutter while in the active mode. The first channel 1443 and the second channel 1445 can be configured to retain the cutter at different angles based on whether the cutter is in the passive mode or active mode. The first channel 1443 can retain the cutter in alignment with (e.g., parallel to) the nosecone, and the second channel 1445 can retain the cutter in alignment with (e.g., parallel to) elongate body. The first distal surface 1415 can be on a protruding lip 1442 on the distal end of the bushing. As described above, the first distal surface 1415 of the bushing can slide along an angled surface a proximal face of the head of the cutter to urge the cutter from the passive mode to the active mode. Also as described above, a surface 1471 of the bushing can slide along an angled surface along the shaft of the cutter to urge the cutter from the active mode to the passive mode.

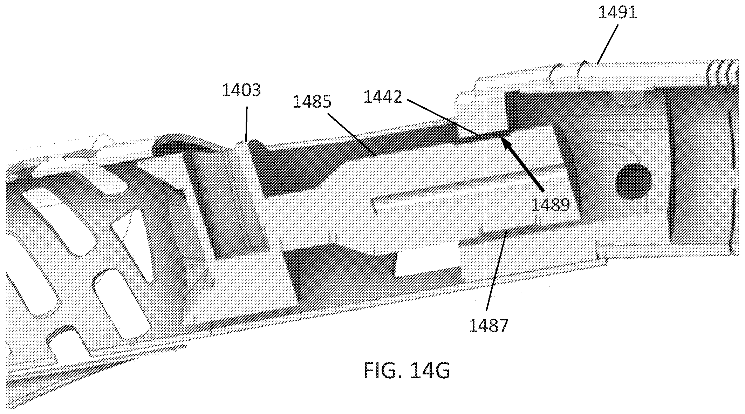

As described above, the cutter 1403 can be retained in the passive mode by detent mechanism. FIG. 14G shows an annular groove 1487 on the shaft 1485 of the cutter 1403, which provides a clearance 1489 (also referred to as a gap) between the cutter and a protruding surface 1442 of the bushing. The clearance 1489 allows the cutter to rotate more freely when the annular groove 1487 is aligned with the internal surface 1442 and the cutting edge of the cutter is housed within the catheter in the passive mode. This configuration can act as a detent to retain the cutter 1403 in the passive mode. For instance, when the cutter is pushed more distally toward the nosecone (e.g., during packing of material into the nosecone) or when the cutter 1403 is pulled more proximally during transition to the active mode, the groove 1487 is not aligned with the internal surface 1442. This causes portions of the shaft 1485 on either side of the groove 1487 to contact the internal surface 1442 of the bushing 1491, thereby increasing a drag (friction) between the cutter 1403 and the bushing 1491. A threshold translational force applied to the cutter 1403 may be required, either in the distal or proximal direction, to release the detent retaining the cutter 1403 in the passive mode.

In some embodiments, any of the atherectomy devices described herein may not include imaging capability.

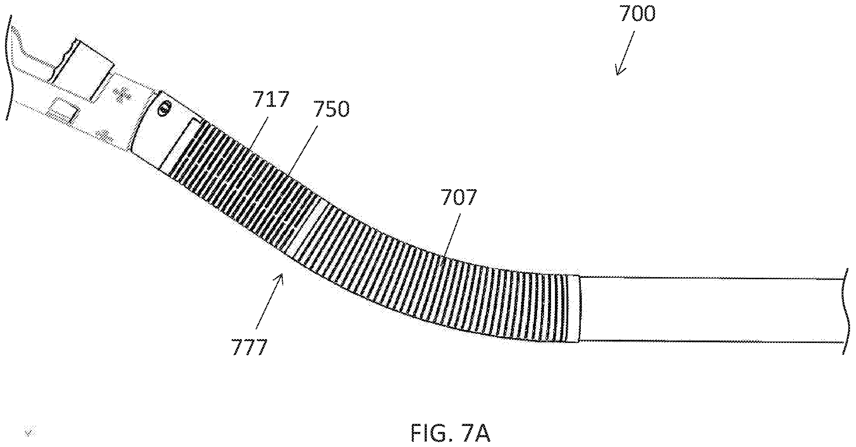

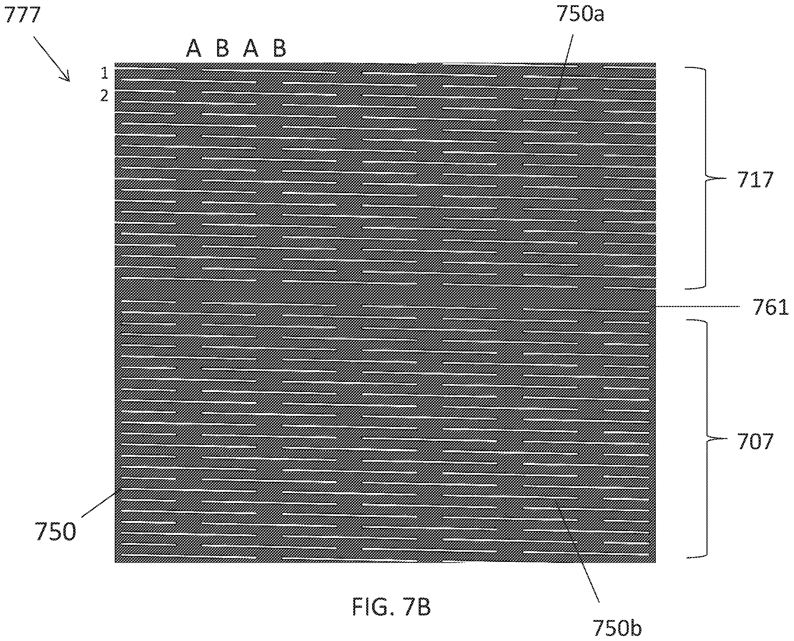

Referring to FIGS. 7A and 7B, in some embodiments, an atherectomy catheter 700 can include a curved portion 777 that includes a fixed jog section 707 and a flexible section 717. The fixed jog section 707 can either be proximal to the flexible section 717 (as shown) or distal to the flexible section 717. In some embodiments, the fixed jog section 707 is longer than the flexible section 717. For example, the fixed jog section 707 can be 5-10mm, such as 8mm, and the flexible section 717 can be 2-6mm, such as 5mm. Further, in some embodiments (as shown), the fixed jog section 707 can include only a single curve rather than a double curve (e.g., forming a c-shape rather than an s-shape). The angle of the curve can be, for example, 120° to 175°, such as 130° to 160°, such as approximately 145°. The flexible section 717 can be configured to bend passively during use (i.e., when acted upon by the vessel wall), for example to form an angle of between 90° and 180°, such as 110-170°, such as 130°-160°.

In some embodiments, the curved portion 777 can be made of a laminated frame. Referring to FIG. 7B, the curved portion 777 can include a frame that includes a plurality of circumferential slits 750a,b extending therethrough. The slits 750a of the flexible section 717 can extend entirely around the circumference (i.e., include no longitudinal spine therein) while the slits 750b of the fixed jog section 707 can end at a longitudinal spine 760 extending through the fixed jog section 707. An annular spine 761 can separate the flexible section 717 and the fixed jog section 707. The frame can be made, for example, of Nitinol or stainless steel. Further, the frame can be laminated with a thin layer of polymer, such as Tecothane, on one or both sides. In some embodiments, only the fixed jog section 707 is laminated while the flexible section 717 remains unlaminated.

Referring to FIG. 7B, the slits 750a,b can be arranged in a pattern that is configured to provide flexibility in the flexible section 717 while maintaining structural integrity of the elongate body in both the flexible section 717 and the fixed jog section 707. Thus, the majority of the slits 750a,b can have the same length, but be offset from one another. For example, the slits 750a in the flexible section 717 can be arranged in rows (1,2) and columns (A, B). Each slit 750a can have a length equivalent to the width of columns A + B + A. Further, the slits can be offset from one another by a distance of A + B. Thus, each column A can include slits from every row 1,2 while column B can include alternating slits (from either row 1 or 2). Column B thus provides structural integrity to the slitted portion of the device. The slits 750a of the flexible section 717 can provide flexibility to allow the catheter 700 to achieve the desired curvature in any direction when inside the body (i.e., the slits can pull apart on the outside of the curve and compress and/or overlap when on the inside of the curve). For example, the flexible section 717 can bend to align the cutter with the edge of the vessel.

Further, the slits 750b in fixed jog section 707 (except the shorter slits bordering the spine 560a) can likewise have a length equivalent to the width of columns A + B + A. Further, the slits can be offset from one another by a distance of A + B. Thus, each column A can include slits from every row 1,2 while column B can include alternating slits (from either row 1 or 2). In fixed jog section 707, however, the spine 760 can be heat-set to set the angle of the jog, fixing the jog.

The curved sections described herein can additionally or alternatively include any of the selective bending support features described in International Application No. PCT/US2019/028415 (the '415 application).

In some embodiments, the selective bending support features described in the '415 application can be modified to take the s-shape as described herein, such as by including spines on opposite sides of the shaft. Additionally, in some embodiments, the selective bending support features described in the '415 application can be modified so as to be activated by compression (e.g., by pulling on the driveshaft of an atherectomy catheter as described herein) rather than via tension.

In some embodiments, the curved portions of the elongate catheter bodies described herein can form a substantially s-shape with two different inflection points of opposite curvatures. In other embodiments, the curved portion can include a single inflection point that forms a substantially C-shape. Further, in some embodiments, one or more of the curves can be fixed. In other embodiments, one or more of the curves can be user activated (e.g., by pulling on the driveshaft or a separate pullshaft or wire). Further, any of the designs described herein can include a flexible section (e.g., of the elongate body or the nosecone) that allows the catheter to take the desired curvature during use.

In some embodiments, the amount of curvature of the user-adjusted curved portions can be further adjusted either prior to or during an atherectomy procedure based on the curvatures of the artery and the location of the plaque formation. For example, by tensioning a shaft of the catheter, the curved portion can constrict and adopt a sharper angle. Alternatively, when the shaft is relaxed, the curved portion can relax and adopt a wider angle. In such examples, the angles of deflection may be adjusted, for example, by 5 to 20 degrees. Further, the shape and angle can be incrementally and/or continuously adjustable, as described herein.

In some embodiments, the user-adjusted curved portions can have a pre-shaped bend or curvature that can be further adjusted prior to or during an atherectomy procedure. In other embodiments, the curved portions can be straight before the user-activated bend is activated.

In any of the embodiments described herein, the nosecone can be configured to hold tissue that is debulked by the cutter. Further, the driveshaft and cutter can be configured to move distally to pack tissue into the nosecone.

In some embodiments, lamination of a framework can cause the laminating material to heat and shrink, pushing into open slits and fixing the shape of the frame (e.g., in a pre-shaped jog). For example, the curved portions 533 and/or 633 can be laminated so as to create a fixed jog that can either be further adjusted by pulling on the driveshaft or that remains fixed throughout the procedure. In other embodiments, lamination of the framework can keep the slits open and free of material, allowing for greater flexibility.

Although described herein as being activated via compression (e.g., pulling on a driveshaft), the curved portions described herein can alternatively be activated via tension (e.g., pushing on a driveshaft).

The atherectomy catheters having a curved portion described herein advantageously allows easier and closer positioning of the atherectomy cutter to plaque close to the inner artery walls. That is, the curved portions can be configured such that the exposed portion of the cutter (e.g., the area extending through the cutter window) moves closer to the vessel wall than the unexposed side of the cutter. This positioning can make cutting during the atherectomy procedure more efficient.

Any of the curved portions described herein may be used alone or in combination with a mechanism to deflect the nosecone. In some embodiments, the nosecone can be deflected by pulling on a cutter driveshaft. Such deflection mechanisms are described in U.S. Patent Application No. 15/072,272, filed March 16, 2016, titled "ATHERECTOMY CATHETERS DEVICES HAVING MULTI-CHANNEL BUSHINGS," now U.S. Patent No. 9,592,075, and U.S. Patent Application No. 15/076,568 filed March 21, 2016, titled "ATHERECTOMY CATHETERS AND OCCLUSION CROSSING DEVICES," now U.S. Patent No. 9/498,247. In some embodiments, placing further tension on the drive shaft (i.e., after exposing the nosecone) can result in compression being applied to the curved portion, causing the curved portion to assume its final curved configuration. Having both the nosecone deflect and the curved portion can result in better tissue invagination and thus better or more efficient tissue cutting.

In embodiments where the nosecone is not deflected, the respective cutting windows can be optimized so as to allow for automatic invagination of tissue into the cutting window. Further, having the nosecone not deflect and relying entirely on the curved portion for tissue apposition can advantageously prevent the cutter from escaping from the nosecone during packing. Further, having the curved portion alone (i.e., without the nosecone activation) can advantageously eliminate having to use additional mechanisms to force a jog mid-surgery, such as pulling or pushing on a shaft, thereby enhancing both ease of use and enhancing image stability.

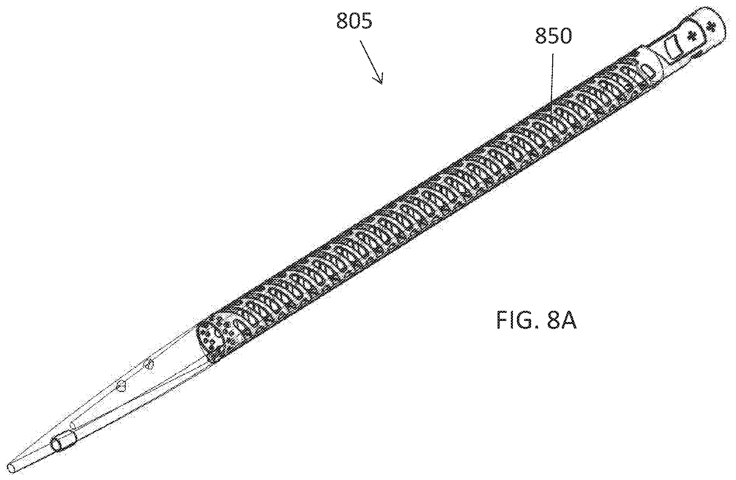

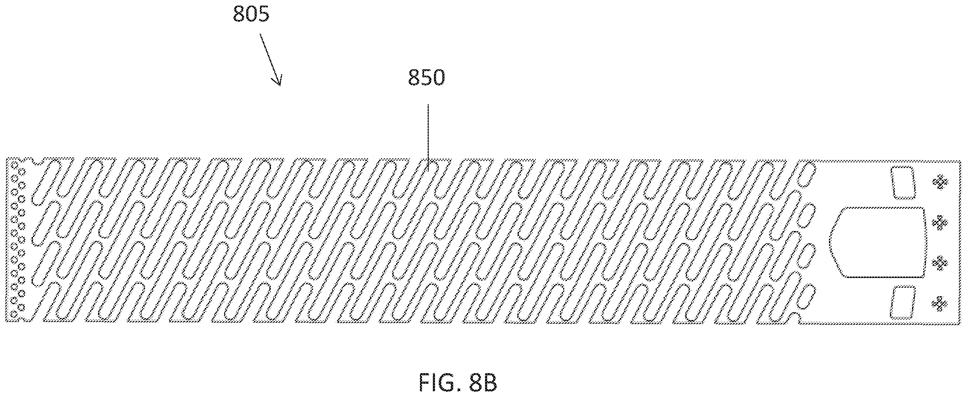

Referring to FIG. 8A, in some embodiments, the nosecone 805 can be flexible. That is, the elongate body can include one or more curves (as described herein), and the nosecone 805 can provide additional flexibility to allow the catheter to take the desired shape. The nosecone 805 can, for example, include a repeating laser cut pattern covered in a laminate layer. As shown in FIG. 8A, the pattern can include a series of spiral slits 850 extending around the circumference of the nosecone. In some embodiments, the laser cut pattern can be cut out of stainless steel, which can be laminated with a polymer, such as Tecothane. Additional flexible nosecone designs are described in U.S. Patent Application No. 14/776,749, filed September 15, 2015 titled "TISSUE COLLECTION DEVICE FOR CATHETER," now U.S. Patent Application Publication No. 2016-0008025-A1 and International Patent Application No. PCT/US2017/035510, filed June 1, 2017 and titled "CATHETER DEVICE WITH DETACHABLE DISTAL END". The flexible nosecone can be used in addition to, or in place of, any feature of the elongate body curved portions described herein.

Any of the catheter devices described herein can include an imaging system for collecting images outside of the catheter. In some embodiments, the imaging system includes a side-facing optical coherence tomography (OTC) system coupled to a cutter and driveshaft for collecting images outside of catheter while the cutter and driveshaft are rotating. Example suitable imaging systems are described in International Application Nos. PCT/US2017/040431 and PCT/US2019/028415.

Any of the catheter devices described herein can include a lock assembly for locking an axial position of the driveshaft (inner shaft) relative to the outer shaft (catheter). The lock assembly can be used, for example, to maintain the catheter distal assembly in a curved or straight state or to keep the cutter positioned outside or inside of the cutter window. In some cases, the lock assembly allows for continuous adjustment of the curvature of the catheter as described herein. In some examples, the locking mechanism is in the handle of the catheter device. FIGS. 15A-15C show an example of a slider lock 1501 in a handle 1500 of the catheter device. In FIG. 15A, the slider lock 1501 is in a distal position where a slider button 1503 is most distally positioned. The inner driveshaft includes a hypotube 1513 and a drive key 1515. The drive key 1515 has a square cross-sectional shape that fits within a correspondingly square shaped opening of a distal end of a cradle 1520. This configuration forms a spline joint assembly 1521 that rotationally couples the drive key 1515 to the cradle 1520 but also allows for axial translation of the drive key 1515 relative to the cradle 1520. Thus, when the cradle 1520 is rotated by the drive motor, the inner driveshaft (drive key 1515 and the hypotube 1513) also rotates, while the spline joint assembly 1521 allows for axial translation of the inner driveshaft (drive key 1515 and the hypotube 1513) relative to the cradle 1520 and the drive motor. A connector piece 1510 is positioned around the drive key 1515 and is used translationally couple the slider button 1503 to the drive key 1515 while allowing the drive key 1515 to rotate independently of the slider button 1503. The connector piece 1510 includes a bearing 1511 (e.g., ball bearing) that allows the drive key 1515 to rotate within the connector piece 1510. The slider button 1503 is rigidly coupled to the connector piece 1510. Thus, distal and proximal movement of the slider button 1503 causes corresponding distal and proximal movement of the inner driveshaft (drive key 1515 and the hypotube 1513). In this way, the spine joint 1521 allows axial translation of the slider button with respect to the driveshaft while allowing the driveshaft to rotate with respect to the other parts of the handle assembly including the slider button.

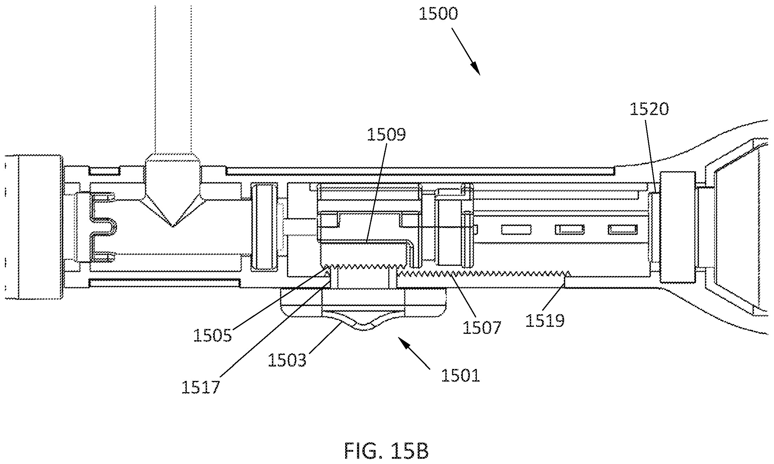

A curved disc spring 1509 provides resistance against the slider button 1503 to keep teeth 1505 of the slider button 1503 engaged with corresponding teeth 1507 within the housing of the handle 1500, thereby locking an axial position of the driveshaft in place. To move the slider button 1503, a user presses the slider button 1503 radially inward to compress the disc spring 1509 and cause the teeth 1505 of the slider button 1503 to disengage from the teeth 1507 within the housing of the handle 1500, as shown in FIG. 15B. When the slider button 1503 is pressed, the user can slide the slider button 1503 proximally or distally. The slider button 1503 is positioned within an opening of the housing of the handle 1500, where the opening is defined by a distal edge 1517 and a proximal edge 1519 that limit the distal and proximal movement of the slider button 1503. Thus, when the user presses on the slider button 1503, the user can translate the slider button 1503 by any distance between the distal edge 1517 and the proximal edge 1519. When the user releases pressure from the slider button 1503, the disc spring 1509 applies pressure back on the slider button 1503 to reengage the teeth 1505 of the slider button 1503 with the teeth 1507 of the housing of the handle 1500, thereby relocking a position of the driveshaft relative to the non-translating parts of the catheter assembly, including the outer shaft. A user can choose an extent to which the driveshaft is translated within the outer shaft as long as the translation of slider button 1503 is between the distal edge 1517 and the proximal edge 1519. The distance between the distal edge 1517 and the proximal edge 1519 can vary depending on design requirements. In some examples, the distance between distal edge 1517 and the proximal edge 1519 ranges from about 0.5 inches to about 1.5 inches (e.g., 0.5-1.5 inches, 0.5-1.0 inches, 0.5-0.75 inches, or 0.75-1.5 inches, or 0.75-1.0 inches). The pitch of the teeth 1505 and 1507 is associated with a fineness of control that the user has for locking the position of the driveshaft. In some examples, the pitch of the teeth 1505 and 1507 is about 0.030 inches or less (e.g., 0.030 inches, 0.028 inches, 0.026 inches, 0.025 inches, 0.023 inches, 0.020 inches, 0.016 inches, or 0.015 inches or less). In some examples, the pitch of the teeth 1505 and 1507 ranges from about 0.020 inches to about 0.040 inches (e.g., 0.020-0.040 inches, 0.025-0.030 inches, or 0.025-0.040 inches).

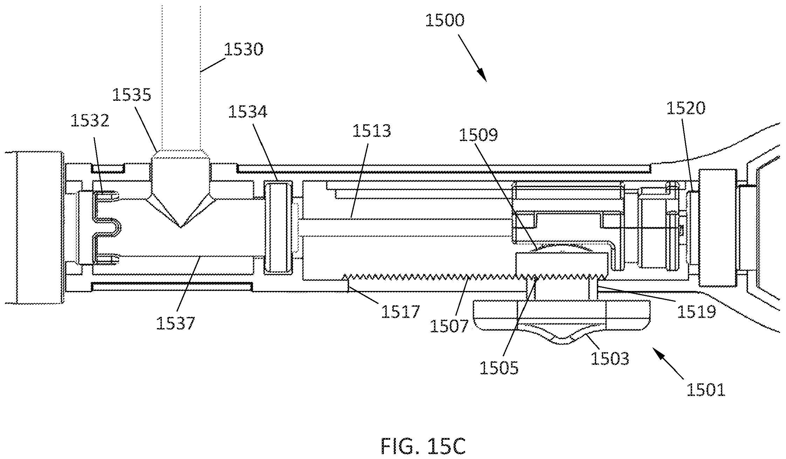

FIG. 15C shows the slider lock 1501 locked in a most proximal position where the slider button 1503 is against the proximal edge 1519 of the housing of the handle 1500. As shown, the teeth 1505 of the slider button 1503 are reengaged with the teeth 1507 within the housing of the handle 1500, thereby locking the axial position of the driveshaft (drive key 1515 and the hypotube 1513) relative to other parts of the catheter system, including the outer shaft. Thus, the slider lock 1501 allows the user to move the driveshaft with respect to the outer shaft while the driveshaft is rotating, as well as allowing the user to choose an extent to which the driveshaft is translated within and locked with respect to the outer shaft. In addition, the slider button 1503 allows a selected axial position of the driveshaft to be locked relative to the outer shaft. These features allow the user to select the extent of curvature of the flexible section of the catheter and to lock the flexible section in selected curvature. These features also allow the user to lock the cutter in an active mode (where the cutter extends through the cutter window) or in a passive mode (where the cutter is retracted within the cutter window). For example, having the slider button 1503 in the distal-most position (e.g., FIG. 15A) can equate to the cutter being in a passive position and the flexible section of the elongate body in a straight or unbent position. Moving the slider button 1503 proximally a little from this most-distal position can cause the cutter to pop out of the cutter window. Moving the slider button 1503 still further proximally can cause the flexible section to bend (e.g., in the s-shape curve) with increasing proximal movement of the slider button 1503 causing increasing amounts of curvature. Having the slider button 1503 in the proximal-most position (e.g., FIG. 15C) can equate to the cutter being in an active position and the flexible section of the elongate body being in a maximally curved position. Moving the slider button 1503 distally from this most-proximal position can cause the flexible section to decrease in curvature with increasing distal movement of the slider button 1503 causing decreasing amounts of curvature. Moving the slider button 1503 still further distally can cause the cutter to retract within the cutter window.

FIG. 15C also illustrates portions of a saline flushing system of the catheter. The handle 1500 can include a connector 1535 that is connected to a tube 1503 to provide fluid (e.g., saline) within portions of the catheter. Although not shown, the flexible tubing 1530 can extend (upward in FIG. 15C) to a connector (e.g., luer connector) that allows the user to directly connect a fluid source (e.g., syringe or saline bag). The fluid can flow through the connector 1535 to a fluid housing 1537, which provides fluidic access between the driveshaft and the outer shaft. The fluid can serve several purposes. For example, the fluid can purge air from the catheter, provide lubrication for the rotational movement of the driveshaft, and can displace blood with an optically transparent fluid (e.g., saline) at a distal end of the catheter so that the imaging system can capture images outside of the catheter while in the blood vessel. In this example, the fluid housing 1537 includes a distal end 1532 with a first seal (e.g., O-ring) and a proximal end 1534 with a second seal (e.g., O-ring) that prevent the fluid from entering other regions of the catheter handle assembly, such as the slider lock 1501 region of the handle 1500.

It should be understood that any features described herein with respect to one embodiment can be combined with or substituted for any feature described herein with respect to another embodiment.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical range recited herein is intended to include all sub-ranges subsumed therein.

Although various illustrative embodiments are described above, any of a number of changes may be made to various embodiments without departing from the scope of the invention as described by the claims. Optional features of various device and system embodiments may be included in some embodiments and not in others. Therefore, the foregoing description is provided primarily for exemplary purposes and should not be interpreted to limit the scope of the invention which is solely set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. An atherectomy device comprising:
a catheter including a distal nosecone (1305) fixedly coupled to a flexible section (1333) by a bushing (1391, 1491) that defines a fixed bend (1325) of the catheter, wherein the catheter includes a cutter window (1307) distal to the fixed bend (1325), wherein the flexible section (1333) includes a longitudinal spine (560a, 560b, 660a, 660b, 960a, 960b, 1160a, or 1160b) on a side of the flexible section (1333); and
a cutter (1303, 1403) coupled to a rotatable driveshaft within the catheter,
wherein proximal movement of the cutter (1303, 1403) and the rotatable driveshaft causes the cutter (1303, 1403) to slide along an edge (1315, 1415) of the bushing (1391, 1491) to cause the cutter (1303, 1403) to tilt in a first direction and to extend through the cutter window (1307),
wherein, when the cutter (1303, 1403) is extended through the cutter window (1307), a force applied to the rotatable shaft in a proximal direction places compression on the flexible section (1333) that causes the flexible section (1333) to bend away from the longitudinal spine (560a, 560b, 660a, 660b, 960a, 960b, 1160a, or 1160b) and to take on a curvature, and
wherein distal movement of the cutter (1303, 1403) and the rotatable driveshaft causes the cutter (1303, 1403) to tilt in a second direction opposite the first direction and to retract within the cutter window (1307).

2. The atherectomy device of claim 1, wherein an extent of curvature of the flexible section (1333) is adjustable based on an amount of force applied to the cutter (1303, 1403) and the rotatable driveshaft in a proximal direction.

3. The atherectomy device of claim 1, wherein the proximal flexible section (1333) is configured to bend to an s-shape.

4. The atherectomy device of claim 1, wherein the flexible section (1333) is configured to bend incrementally based on an amount of compression on the flexible section (1333) via proximal movement of the cutter (1303, 1403) and the rotatable driveshaft.

5. The atherectomy device of claim 1, wherein the cutter (1303, 1403) includes a ledge (1311) that is configured to slide along the edge (1315, 1415) of the bushing (1391, 1491) to move the cutter (1303, 1403) radially and extend the cutter (1303, 1403) through the cutter window (1307).

6. The atherectomy device of claim 4, wherein the edge (1315, 1415) is sloped with respect to an axis perpendicular to the longitudinal axis of the distal nosecone (1305).

7. The atherectomy device of claim 4, wherein the edge (1315, 1415) is configured to tilt the cutter (1303, 1403) with respect to the nosecone (1305) when the ledge (1311) of the cutter (1303, 1403) slides along the edge (1315, 1415).

8. The atherectomy device of claim 1, wherein the bushing (1391) includes an internal edge (1471) that is configured to slide along an angled surface (1470) of the cutter (1303, 1403) to cause the cutter (1303, 1403) to tilt in the second direction and retract within the cutter window (1307).

9. The atherectomy device of claim 1, further comprising a handle (1500) at a proximal end of the catheter, wherein the handle includes a lock (1501) configured to lock the flexible section (1333) in a curved shape having a selected extent of curvature.

10. The atherectomy device of claim 9, wherein the lock (1501) is configured to allow a user to choose a locked position of the driveshaft with respect to the catheter by 0.026 inches or less.

11. The atherectomy device of claim 9, wherein the lock (1501) includes a slider button (1503) that is configured to slide distally and proximally.

12. The atherectomy device of claim 11, wherein sliding the slider button (1503) proximally increases the curvature of the flexible section (1333).

13. The atherectomy device of claim 11, wherein the slider button (1503) includes teeth (1505) that are configured to engage with corresponding teeth (1507) within the handle (1500) to lock an axial position of the driveshaft with respect to the catheter.

14. The atherectomy device of claim 13, wherein the handle (1500) includes a spring (1509) that applies pressure to the slider button (1503) to keep the teeth (1505) of the slider button (1503) engaged with the corresponding teeth (1507) within the handle (1500).

15. The atherectomy device of claim 13, wherein the slider button (1503) is configured to compress the spring (1509) when a user presses on the slider button (1503) to disengage the teeth (1505) of the slider button (1509) from the corresponding teeth (1507) within the handle (1500).

## Patentansprüche

1. Atherektomievorrichtung, umfassend:
einen Katheter, der einen distalen Nasenkonus (1305) einschließt, der fest mit einem flexiblen Abschnitt (1333) durch eine Buchse (1391, 1491) verbunden ist, die eine feste Biegung (1325) des Katheters definiert, wobei der Katheter ein Schneidfenster (1307) distal zu der festen Biegung (1325) einschließt, wobei der flexible Abschnitt (1333) einen Längsstachel (560a, 560b, 660a, 660b, 960a, 960b, 1160a oder 1160b) auf einer Seite des flexiblen Abschnitts (1333) einschließt; und
ein Schneidwerkzeug (1303, 1403), das mit einer drehbaren Antriebswelle innerhalb des Katheters gekoppelt ist,
wobei eine proximale Bewegung des Schneidwerkzeugs (1303, 1403) und der drehbaren Antriebswelle bewirkt, dass das Schneidwerkzeug (1303, 1403) entlang eines Rands (1315, 1415) der Buchse (1391, 1491) gleitet, um zu bewirken, dass das Schneidwerkzeug (1303, 1403) in eine erste Richtung kippt und sich durch das Schneidwerkzeugfenster (1307) erstreckt,
wobei, wenn das Schneidwerkzeug (1303, 1403) durch das Schneidwerkzeugfenster (1307) ausgefahren wird, eine auf die drehbare Welle in einer proximalen Richtung ausgeübte Kraft den flexiblen Abschnitt (1333) komprimiert, was den flexiblen Abschnitt (1333) dazu veranlasst, sich vom Längsstachel (560a, 560b, 660a, 660b, 960a, 960b, 1160a oder 1160b) wegzubiegen und eine Krümmung anzunehmen, und wobei eine distale Bewegung des Schneidwerkzeugs (1303, 1403) und der drehbaren Antriebswelle bewirkt, dass das Schneidwerkzeug (1303, 1403) in eine zweite, der ersten Richtung entgegengesetzte Richtung kippt und sich in das Schneidwerkzeugfenster (1307) zurückzieht.

2. Atherektomievorrichtung nach Anspruch 1, wobei ein Ausmaß der Krümmung des flexiblen Abschnitts (1333) basierend auf einer auf das Schneidwerkzeug (1303, 1403) und die drehbare Antriebswelle in einer proximalen Richtung ausgeübten Kraft einstellbar ist.

3. Atherektomievorrichtung nach Anspruch 1, wobei der proximale flexible Abschnitt (1333) konfiguriert ist, um sich in eine S-Form zu biegen.

4. Atherektomievorrichtung nach Anspruch 1, wobei der flexible Abschnitt (1333) konfiguriert ist, um sich basierend auf dem Ausmaß der Komprimierung des flexiblen Abschnitts (1333) über eine proximale Bewegung des Schneidwerkzeugs (1303, 1403) und der drehbaren Antriebswelle schrittweise zu biegen.

5. Atherektomievorrichtung nach Anspruch 1, wobei das Schneidwerkzeug (1303, 1403) eine Leiste (1311) einschließt, die konfiguriert ist, um entlang des Rands (1315, 1415) der Buchse (1391, 1491) zu gleiten, um das Schneidwerkzeug (1303, 1403) radial zu bewegen und das Schneidwerkzeug (1303, 1403) durch das Schneidwerkzeugfenster (1307) zu verlängern.

6. Atherektomievorrichtung nach Anspruch 4, wobei der Rand (1315, 1415) in Bezug auf eine Achse, die senkrecht zur Längsachse des distalen Nasenkonus (1305) verläuft, geneigt ist.

7. Atherektomievorrichtung nach Anspruch 4, wobei der Rand (1315, 1415) konfiguriert ist, um das Schneidwerkzeug (1303, 1403) in Bezug auf den Nasenkonus (1305) zu neigen, wenn die Leiste (1311) des Schneidwerkzeugs (1303, 1403) entlang des Rands (1315, 1415) gleitet.

8. Atherektomievorrichtung nach Anspruch 1, wobei die Buchse (1391) einen inneren Rand (1471) einschließt, der konfiguriert ist, um entlang einer abgewinkelten Oberfläche (1470) des Schneidwerkzeugs (1303, 1403) zu gleiten, um das Schneidwerkzeug (1303, 1403) zu veranlassen, sich in die zweite Richtung zu neigen und sich in das Schneidwerkzeugfenster (1307) zurückzuziehen.

9. Atherektomievorrichtung nach Anspruch 1, ferner umfassend einen Griff (1500) an einem proximalen Ende des Katheters, wobei der Griff einen Verschluss (1501) aufweist, der konfiguriert ist, um den flexiblen Abschnitt (1333) in einer gekrümmten Form mit einem ausgewählten Ausmaß an Krümmung zu verriegeln.

10. Atherektomievorrichtung nach Anspruch 9, wobei der Verschluss (1501) konfiguriert ist, um es einem Benutzer zu ermöglichen, eine verriegelte Position der Antriebswelle in Bezug auf den Katheter um 0,026 Zoll oder weniger zu wählen.

11. Atherektomievorrichtung nach Anspruch 9, wobei der Verschluss (1501) eine Gleitertaste (1503) einschließt, die konfiguriert ist, um distal und proximal zu gleiten.

12. Atherektomievorrichtung nach Anspruch 11, wobei das Verschieben der Gleitertaste (1503) in proximaler Richtung die Krümmung des flexiblen Abschnitts (1333) erhöht.

13. Atherektomievorrichtung nach Anspruch 11, wobei die Gleitertaste (1503) Zähne (1505) einschließt, die konfiguriert sind, um mit entsprechenden Zähnen (1507) innerhalb des Griffs (1500) in Eingriff zu kommen, um eine axiale Position der Antriebswelle in Bezug auf den Katheter zu sperren.

14. Atherektomievorrichtung nach Anspruch 13, wobei der Griff (1500) eine Feder (1509) einschließt, die Druck auf die Gleitertaste (1503) ausübt, um die Zähne (1505) der Gleitertaste (1503) in Eingriff mit den entsprechenden Zähnen (1507) innerhalb des Griffs (1500) zu halten.

15. Atherektomievorrichtung nach Anspruch 13, wobei die Gleitertaste (1503) konfiguriert ist, um die Feder (1509) zu komprimieren, wenn ein Benutzer auf die Gleitertaste (1503) drückt, um die Zähne (1505) der Gleitertaste (1509) von den entsprechenden Zähnen (1507) innerhalb des Griffs (1500) zu lösen.

## Revendications

1. Dispositif d'athérectomie comprenant :
un cathéter comprenant un embout nasal distal (1305) couplé fixement à une section flexible (1333) par une douille (1391, 1491) qui définit une courbure fixe (1325) du cathéter, dans lequel le cathéter comprend une fenêtre de coupe (1307) distale par rapport à la courbure fixe (1325), dans lequel la section flexible (1333) comprend une épine longitudinale (560a, 560b, 660a, 660b, 960a, 960b, 1160a, ou 1160b) sur un côté de la section flexible (1333) ; et
un couteau (1303, 1403) couplé à un arbre d'entraînement rotatif à l'intérieur du cathéter,
dans lequel un mouvement proximal du couteau (1303, 1403) et de l'arbre d'entraînement rotatif amène le couteau (1303, 1403) à glisser le long d'un bord (1315, 1415) de la douille (1391, 1491) pour amener le couteau (1303, 1403) à s'incliner dans une première direction et à s'étendre à travers la fenêtre du couteau (1307),
dans lequel, lorsque le couteau (1303, 1403) est étendu à travers la fenêtre de couteau (1307), une force appliquée à l'arbre rotatif dans une direction proximale exerce une compression sur la section flexible (1333) qui amène la section flexible (1333) à se plier en s'éloignant de l'épine longitudinale (560a, 560b, 660a, 660b, 960a, 960b, 1160a, ou 1160b) et à prendre une courbure, et
dans lequel un mouvement distal du couteau (1303, 1403) et de l'arbre d'entraînement rotatif amène le couteau (1303, 1403) à s'incliner dans une seconde direction opposée à la première direction et à se rétracter dans la fenêtre du couteau (1307).

2. Dispositif d'athérectomie selon la revendication 1, dans lequel un niveau de courbure de la section flexible (1333) est réglable sur la base d'une quantité de force appliquée au couteau (1303, 1403) et à l'arbre d'entraînement rotatif dans une direction proximale.

3. Dispositif d'athérectomie selon la revendication 1, dans lequel la section flexible proximale (1333) est configurée pour se plier en forme de S.

4. Dispositif d'athérectomie selon la revendication 1, dans lequel la section flexible (1333) est configurée pour se plier de manière incrémentielle sur la base d'une quantité de compression sur la section flexible (1333) via un mouvement proximal du couteau (1303, 1403) et de l'arbre d'entraînement rotatif.

5. Dispositif d'athérectomie selon la revendication 1, dans lequel le couteau (1303, 1403) comprend un rebord (1311) qui est configuré pour coulisser le long du bord (1315, 1415) de la douille (1391, 1491) pour déplacer le couteau (1303, 1403) radialement et étendre le couteau (1303, 1403) à travers la fenêtre de couteau (1307).

6. Dispositif d'athérectomie selon la revendication 4, dans lequel le bord (1315, 1415) est incliné par rapport à un axe perpendiculaire à l'axe longitudinal du cône nasal distal (1305).

7. Dispositif d'athérectomie selon la revendication 4, dans lequel le bord (1315, 1415) est configuré pour incliner le couteau (1303, 1403) par rapport au cône nasal (1305) lorsque le rebord (1311) du couteau (1303, 1403) glisse le long du bord (1315, 1415).

8. Dispositif d'athérectomie selon la revendication 1, dans lequel la douille (1391) comprend un bord interne (1471) qui est configuré pour glisser le long d'une surface inclinée (1470) du couteau (1303, 1403) pour amener le couteau (1303, 1403) à s'incliner dans la seconde direction et à se rétracter dans la fenêtre de couteau (1307).

9. Dispositif d'athérectomie selon la revendication 1, comprenant en outre une poignée (1500) à une extrémité proximale du cathéter, dans lequel la poignée comprend un verrou (1501) configuré pour verrouiller la section flexible (1333) dans une forme incurvée ayant un niveau de courbure sélectionné.

10. Dispositif d'athérectomie selon la revendication 9, dans lequel le verrou (1501) est configuré pour permettre à un utilisateur de choisir une position verrouillée de l'arbre d'entraînement par rapport au cathéter de 0,026 pouce ou moins.

11. Dispositif d'athérectomie selon la revendication 9, dans lequel le verrou (1501) comprend un bouton coulissant (1503) qui est configuré pour coulisser de manière distale et proximale.

12. Dispositif d'athérectomie selon la revendication 11, dans lequel le glissement du bouton coulissant (1503) de manière proximale augmente la courbure de la section flexible (1333).

13. Dispositif d'athérectomie selon la revendication 11, dans lequel le bouton coulissant (1503) comprend des dents (1505) qui sont configurées pour s'engager avec des dents correspondantes (1507) à l'intérieur de la poignée (1500) pour verrouiller une position axiale de l'arbre d'entraînement par rapport au cathéter.

14. Dispositif d'athérectomie selon la revendication 13, dans lequel la poignée (1500) comprend un ressort (1509) qui applique une pression sur le bouton coulissant (1503) pour maintenir les dents (1505) du bouton coulissant (1503) engagées avec les dents correspondantes (1507) à l'intérieur de la poignée (1500).

15. Dispositif d'athérectomie selon la revendication 13, dans lequel le bouton coulissant (1503) est configuré pour comprimer le ressort (1509) lorsqu'un utilisateur appuie sur le bouton coulissant (1503) pour désengager les dents (1505) du bouton coulissant (1509) des dents correspondantes (1507) à l'intérieur de la poignée (1500).
